(19) Europäisches Patentamt / European Patent Office / Office européen des brevets

(11) **EP 1 795 606 A1**

(12) **EUROPEAN PATENT APPLICATION**

(43) Date of publication:
**13.06.2007 Bulletin 2007/24**

(51) Int Cl.:
***C12Q 1/37*** (2006.01)

(21) Application number: **05017523.1**

(22) Date of filing: **11.08.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **DIGILAB BioVisioN GmbH**
**30625 Hannover (DE)**

(72) Inventors:
• **Budde, Petra**
  **30655 Hannover (DE)**
• **Schulz-Knappe, Peter**
  **30966 Hemmingen (DE)**

(74) Representative: **Schnappauf, Georg et al**
**Dr. Volker Vossius**
**Patent- und Rechtsanwaltskanzlei**
**Geibelstrasse 6**
**81679 München (DE)**

(54) **Method for screening for proteases and their substrates**

(57) The invention relates to methods for screening for proteases and substrates susceptible to cleavage by proteases. The methods of the invention allow for the screening of proteases in an environment where the proteases are normally present, in particular within a living organism. By inhibiting the activity of at least one protease, preferably at least one first-line protease for a given substrate, which usually produces the majority of proteolytic fragments derived from said substrate and thus, would normally (without inhibiting said activity) mask the activity of a second-line protease, it is possible to identify proteases that perform second-line proteolysis and their recognition sequences.

EP 1 795 606 A1

SEQUENCE LISTING

```
<110>  BioVision AG

<120>  METHOD FOR SCREENING FOR PROTEASES AND THEIR SUBSTRATES

<130>  412-34

<160>  4

<170>  PatentIn version 3.1

<210>  1
<211>  30
<212>  PRT
<213>  Homo sapiens

<400>  1

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg
            20              25              30


<210>  2
<211>  13
<212>  PRT
<213>  Homo sapiens

<400>  2

His Ala Glu Gly Thr Phe Thr Ser Asp Val Ser Ser Tyr
1               5                   10


<210>  3
<211>  17
<212>  PRT
<213>  Homo sapiens

<400>  3

Leu Glu Gly Gln Ala Ala Lys Glu Phe Ile Ala Trp Leu Val Lys Gly
1               5                   10                  15


Arg


<210>  4
<211>  28
<212>  PRT
<213>  Homo sapiens

<400>  4

Glu Gly Thr Phe Thr Ser Asp Val Ser Ser Tyr Leu Glu Gly Gln Ala
1               5                   10                  15
```

```
Ala Lys Glu Phe Ile Ala Trp Leu Val Lys Gly Arg
              20                    25
```

**Description**

**Field of the invention**

The present invention is in the field of pharmaceutical research tools. Specifically, the present invention provides methods for screening for proteases and substrates susceptible to cleavage by proteases.

**Background of the invention**

It is known that proteases play important roles in normal cell functions and in diseases. They are targets for certain drugs in the treatment of, for example, cancers, parasitic, fungal and viral infections, and inflammatory, immunological, respiratory, cardiovascular and neurodegenerative disorders (Leung et al., J. Med. Chem. 43: 305-41, 2000). It is estimated that the human genome encoded between 1000 and 2000 proteases, among which many will be potential targets for drug discovery projects (Southan, Drug Discov. Today 6: 681-8, 2001; Southan, FEBS Lett. 498: 214-8, 2001). Protease regulators, especially protease inhibitors, are strongly desired for their potential therapeutic applications, especially in the treatment of cancer, high blood pressure and diabetes, but also other medical indications such as Alzheimer's Disease, disorders of the coagulation cascade such as deep vein thrombosis, pulmonary embolism, myocardial infarction, stroke, and disseminated intravascular coagulation during sepsis. Furthermore several infectious diseases such as HIV-infections are successfully treatable with protease inhibitors, pointing to the importance of this class of therapeutic molecules also in the field of infectious diseases. Proteases cleaving physiologically important peptides or proteins are attractive drug targets. An example of such a pair of a protease and physiologically important substrate cleaved by said protease are, for example, dipeptidylpeptidase 4 (DPP4) which cleaves the glucacon-like peptide 1 (GLP-1).

Accordingly, there is a need for an effective and sensitive screening method for the identification of candidate proteases having the ability to cleave physiologically important peptides or proteins and for the identification of those amino acid sequences representing protease recognition sequences for said proteases.

There are two main aspects where protection of substrates from proteolytic cleavage is of therapeutic benefit:

In one aspect, the protease substrates, in particular peptides and/or proteins, have valuable biological activity that needs to be preserved in order to reach a therapeutic goal. One example is the peptide hormone GLP-1 which is useful in the treatment of diabetes. The peak plasma concentrations (and presumably tissue concentrations as well) and half-life thereof can be increased by inhibiting DPP4, a protease known to cleave GLP-1 into an inactive form.

In a second aspect, the products that result from the action of a protease on a particular substrate are harmful to an individual, and therefore, their neo-generation needs to be blocked in order to achieve a therapeutic effect. One example is angiotensin, that increases blood pressure to a harmful extent in patients with (renal) hypertension. Its production by the protease angiotensin-converting enzyme (ACE) is blocked by ACE-inhibitors and thus, peak plasma concentrations are lowered, while half-life is not affected.

Two different strategies may be used to address the above aspects:

Either (i) the protease that cleaves the substrate is inhibited, e.g. by means of protease inhibitors, or, (ii) the sequence area of the substrate that is susceptible to cleavage by a protease is protected against cleavage. This includes, for example, chemical modification and amino acid substitution.

Inhibition of a protease may increase the biological half-life of the substrate. However, it may also be that this effect is reduced or compensated due to the fact that other proteases are or become responsible for the cleavage of the substrate. The present invention aims at the identification of these proteases (second-line proteases).

Specifically, the present invention generally is directed to methods for the identification of proteases that perform such second-line proteolysis. According to the invention, the activity of second-line proteolysis is detected in a background where the activity of at least one first-line protease is inhibited. The invention offers the possibility to screen for such second-line proteases and to identify the recognition sequences for such second-line proteases.

**Summary of the invention**

The methods according to the invention allow for the screening of proteases in an environment where the proteases are normally present, in particular within a living organism. By inhibiting the activity of at least one protease, preferably at least one first-line protease for a given substrate, which usually produces the majority of proteolytic fragments derived from said substrate and thus, would normally (without inhibiting said activity) mask the activity of a second-line protease, it is possible to identify proteases that perform second-line proteolysis and their recognition sequences.

The methods of the invention do not require the purification of proteases and therefore, do not have the risk of changing the activity and/or specificity, in particular inactivating one or more proteases, due to purification procedures. Furthermore, it is possible to test simultaneously more than one protease present in a sample. In addition, the methods of the invention allow for the testing in a natural environment, in particular in vivo in a living organism, especially in a living mammalian organism.

The methods of the present invention may be used to screen for proteases, in particular second-line proteases and their recognition sequences, possibly useful as drug targets. Based on the recognition sequences identified it will be possible to synthesize proteolysis-resistant variants of protease substrates, for example peptidomimetics of these substrates. Based on the second-line proteases identified, it will be possible to design and to screen for protease inhibitors directed at these second-line proteases. It might be that protease inhibitors already exist which are active towards the second-line proteases identified according to the invention.

It will also be possible to combine inhibitors directed against the second-line proteases identified according to the invention with inhibitors for proteases known to cleave the same substrate. This could improve the efficiency of therapeutic approaches, lead to more specific therapeutic strategies and allow to reduce the dosis of the protease inhibitors previously used for therapy.

In a first aspect, the invention relates to a method of determining whether proteolytic activity for a given substrate is present in a biological sample, the method comprising the steps of a) inhibiting in said biological sample or a fraction thereof the activity of at least one protease known to proteolytically cleave said given substrate, b) allowing the proteases present in said biological sample or said fraction thereof to exert proteolytic activity on said given substrate in said biological sample or said fraction thereof, and c) analysing said given substrate and/or proteolytic fragments thereof generated in step b).

In a further aspect, the invention relates to a method for identifying at least one sequence in a substrate which is subject to cleavage by proteases present in a biological sample, the method comprising the steps of a) inhibiting in said biological sample or a fraction thereof the activity of at least one protease known to proteolytically cleave said given substrate, b) allowing the proteases present in said biological sample or said fraction thereof to exert proteolytic activity on said given substrate in said biological sample or said fraction thereof, and c) analysing said given substrate and/or proteolytic fragments thereof generated in step b).

In preferred embodiments of the methods according to these aspects of the invention said given substrate on which proteolytic activity in step (b) is allowed to be exerted is naturally present in said biological sample and/or has been added to said biological sample or said fraction thereof. Addition of a given substrate includes also giving the substrate to an individual, preferably to a mammal.

In still another aspect, the invention relates to a method of determining whether a given sequence is subject to cleavage by proteases present in a biological sample, the method comprising the steps of a) inhibiting in said biological sample or a fraction thereof the activity of at least one protease known to proteolytically cleave a substrate comprising said given sequence or a variant thereof, b) allowing the proteases present in said biological sample or said fraction thereof to exert proteolytic activity on a substrate comprising said given sequence in said biological sample or said fraction thereof, and c) analysing said substrate comprising said given sequence and/or proteolytic fragments thereof generated in step b). Preferably said given sequence is derived from a substrate, preferably a naturally occurring substrate, known to be cleaved by said at least one protease the activity of which is inhibited or known to be cleaved by other proteases present in the biological sample, preferably second-line proteases. Said sequence may correspond to whole or part of said substrate and additionally may be modified compared to the corresponding sequence of said substrate. In one preferred embodiment of the method according to this aspect of the invention the substrate is a non-naturally occurring substrate, i.e. an artificial substrate such as a modified peptide or protein, which has been added to the sample tested. Thus, said method allows for the screening of sequences which are subject to proteolytic cleavage as well as sequences which are resistant to proteolytic cleavage.

In preferred embodiments of the methods according to all aspects of the invention, the substrate on which proteolytic activity is allowed to be exerted comprises at least one label, preferably selected from the group consisting of stable isotope-labels, radioactive isotope-labels, dye-labels, fluorescent labels, FRET-labels, particle-labels, ITRAQ-labels.

Preferably, the step of inhibiting the activity of at least one protease in the methods of the invention comprises an inhibition of the activity of at least one first-line protease, preferably of all first-line proteases for a given substrate or a substrate comprising a given sequence or variant thereof present in a sample and the proteolytic activity is exerted at least in part by at least one second-line protease, wherein said at least one first-line protease and/or said at least one second-line protease may be naturally present in a biological sample. Inhibition of the activity of at least one protease is preferably achieved by means of at least one protease inhibitor for said at least one protease. Thus, the methods of the invention preferably relate to the determination whether proteolytic activity of at least one second-line protease for a given substrate is present in a biological sample, the identification of at least one sequence in a substrate which is subject to cleavage by at least one second-line protease present in a biological sample and the determination whether a given sequence is subject to cleavage by at least one second-line protease present in a biological sample or not.

Preferably the step of analysing a substrate and/or proteolytic fragments thereof in the methods of the invention comprises a qualitative and/or quantitative analysis of said substrate and/or proteolytic fragments thereof. In one preferred embodiment the step of analysing a substrate and/or proteolytic fragments thereof is performed in a time dependent manner, and preferably is performed at a first time and at least a second time.

In a further preferred embodiment, the step of analysing in the methods of the invention includes a determination of at least one full or partial sequence of at least one of the proteolytic fragments generated in the methods of the invention. Said at least one full or partial sequence of said proteolytic fragments is preferably used to determine the sequence of at least one protease recognition sequence for at least one of the proteases present in a biological sample wherein the protease recognition sequence may be used to identify at least one protease responsible for the cleavage of a substrate. Preferably at least one of said at least one protease recognition sequence is a protease recognition sequence for second-line proteases and at least one of said at least one protease identified is a second-line protease.

In preferred embodiments, the methods of the invention further comprise the step of comparing the results obtained in the step of analysing a substrate and/or proteolytic fragments thereof with the results obtained from a reference sample, such as the results obtained from (i) analysing the substrate and/or proteolytic fragments thereof prior to the step of inhibiting the activity of least one protease in the biological sample or fraction thereof or a corresponding biological sample or fraction thereof and/or (ii) analysing the substrate and/or proteolytic fragments thereof in a corresponding biological sample or fraction thereof in which the activity of at least one of said at least one protease is not inhibited and preferably the activity of none of said at-least one protease is inhibited and/or the activity of at least one protease is inhibited which is not inhibited in the test sample and/or (iii) analysing the substrate and/or proteolytic fragments thereof in a corresponding biological sample or fraction thereof in which the activity of at least one of said at least one protease is inhibited to a different extent and/or (iv) analysing the substrate and/or proteolytic fragments thereof in a corresponding biological sample or fraction thereof in which the activity of at least one of said at least one protease is inhibited by different means and/or (v) analysing the substrate and/or proteolytic fragments thereof in a corresponding biological sample or fraction thereof in which the activity of at least one of said at least one protease is inhibited for a different period of time.

In one embodiment, step (a) or steps (a) and (b) of the methods of the invention take place within a living organism. In this embodiment, the results obtained in step (c) of the methods of the invention may be compared with the results obtained from at least one further living organism wherein in said at least one further living organism preferably (i) the activity of at least one of said at least one protease is not inhibited and preferably the activity of none of said at-least one protease is inhibited and/or the activity of at least one protease is inhibited which is not inhibited in said living organism and/or (ii) the activity of at least one of said at least one protease is inhibited to a different extent and/or (iii) the activity of at least one of said at least one protease is inhibited by different means and/or (iv) the activity of at least one of said at least one protease is inhibited for a different period of time.

In preferred embodiments of the methods of the invention, said living organism and said at least one further living organism have been subjected to or are subjected to different environmental conditions, and/or are of different age, sex, weight or health condition. Furthermore, said living organism and said at least one further living organism may be of the same or different species. If said living organism and said at least one further living organism are of the same species they may have different genetic backgrounds. In certain embodiments of the methods of the invention said living organism and/or said at least one further living organism is afflicted with a disease.

In particular embodiments of the methods of the invention said living organism and/or said at least one further living organism is a multicellular organism, preferably a mammal.

In preferred embodiments of the methods of the invention, inhibition of the activity of at least one protease is achieved by means of at least one protease inhibitor for said at least one protease. Preferably, said at least one protease inhibitor is delivered to said living organism and/or said at least one further living organism.

In further preferred embodiments of the methods of the invention the at least one first-line protease and/or the at least one second-line protease comprise at least one protease selected from the group consisting of aspartic peptidases, cysteine peptidases, glutamic peptidases, metallo peptidases, serine peptidases, threonine peptidases, amino peptidases, carboxypeptidases, endopeptidases and exopeptidases. Preferably the first-line protease is dipeptidyl peptidase 4 (DPP4). In this embodiment of the methods of the invention, the substrate is preferably glucagon-like peptide 1 (GLP-1).

In a preferred embodiment of the methods of the invention the step of analysing a substrate and/or proteolytic fragments thereof comprises an analysis of the entire biological sample or fraction thereof, an analysis of all peptides and/or proteins in said biological sample or fraction thereof or an analysis of all proteolytic fragments in said biological sample or fraction thereof or comprises an analysis of only a fraction of said biological sample or fraction thereof, in particular a fraction of said peptides and/or proteins or said proteolytic fragments in said biological sample or said fraction thereof.

Furthermore, the step of analysing a substrate and/or proteolytic fragments thereof preferably comprises a determination of the relative or absolute quantity of said substrate and/or proteolytic fragments thereof. Preferably said step is performed using mass spectrometric methods or mass spectrometric methods in combination with separation methods for said substrate and/or proteolytic fragments thereof. Said separation methods may be performed prior to, simultaneous with and/or subsequent to said mass spectrometric methods or a combination thereof. Preferred separation methods are selected from the group consisting of gel electrophoresis, liquid chromatography, gas chromatography, capillary chromatography, thin layer chromatography, mass spectrometry, precipitation techniques, liquid phase extraction techniques, filtration and other molecular size discriminating techniques. Furthermore preferred mass spectrometric methods are selected from the group consisting of MALDI mass spectrometry, ESI mass spectrometry, SELDI mass spectrometry, FAB mass spectrometry and MS/MS mass spectrometry.

In preferred embodiments of the methods of the invention a biological sample is selected from the group consisting of blood, serum, plasma, urine, tear fluid, saliva, synovia, liquor, bronchial aspirate, sputum, lymph, tissue extracts, cell extracts and cell culture medium.

**Brief description of the drawings**

Figure 1:

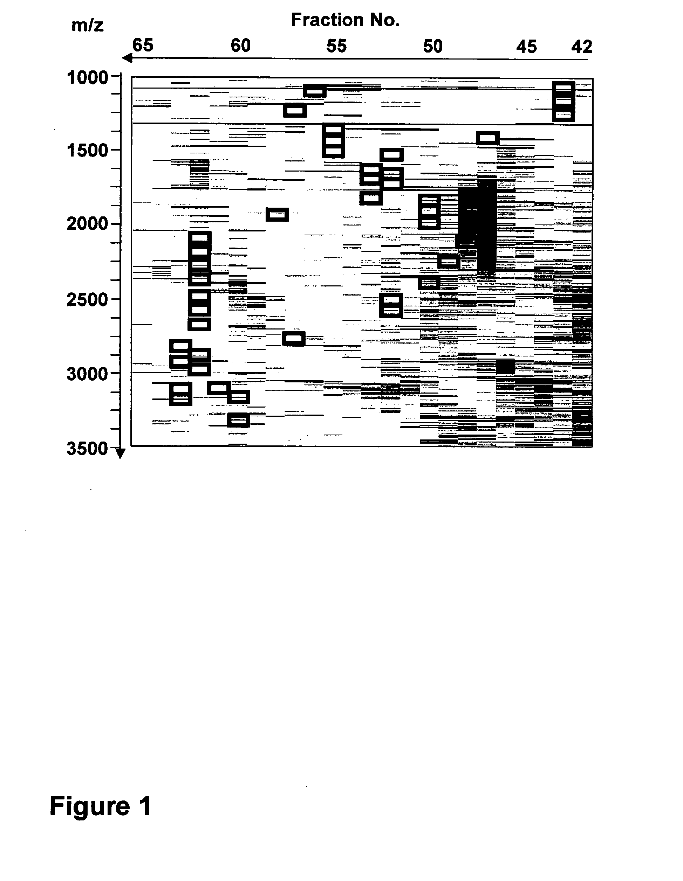

Figure 1 shows a peptide map of rat plasma. All 58 theoretically possible proteolytic fragments of human glucagon-like protein-1, GLP-1 (if GLP-1 is only proteolytically cleaved once) were calculated regarding their molecular mass and amino acid sequence and the fraction numbers were predicted if GLP-1 is only cleaved once per molecule and if these GLP-1 fragments were separated into 96 fractions using reversed phase chromatography (prediction method described in: EP 1553515 A1). The calculated and predicted data resulted in a hypothetic list comprising the corresponding coordinates within a peptide map (molecular mass, fraction number; see Table 1). These coordinates of all theoretically possible proteolytic GLP-1 fragments were superimposed onto a peptide map generated using rat plasma. Each pair of coordinates (molecular mass, fraction number) is indicated by a small box. Extraction from the peptide map of only the data from these coordinates could be used to selectively analyze all potential proteolytic fragments of GLP-1 if each GLP-1 molecule is cleaved only once. The peptide map used for this figure is only exemplarily and is not from a rat treated with human GLP-1 or treated with a DPP4-protease inhibitor.

Figure 2:

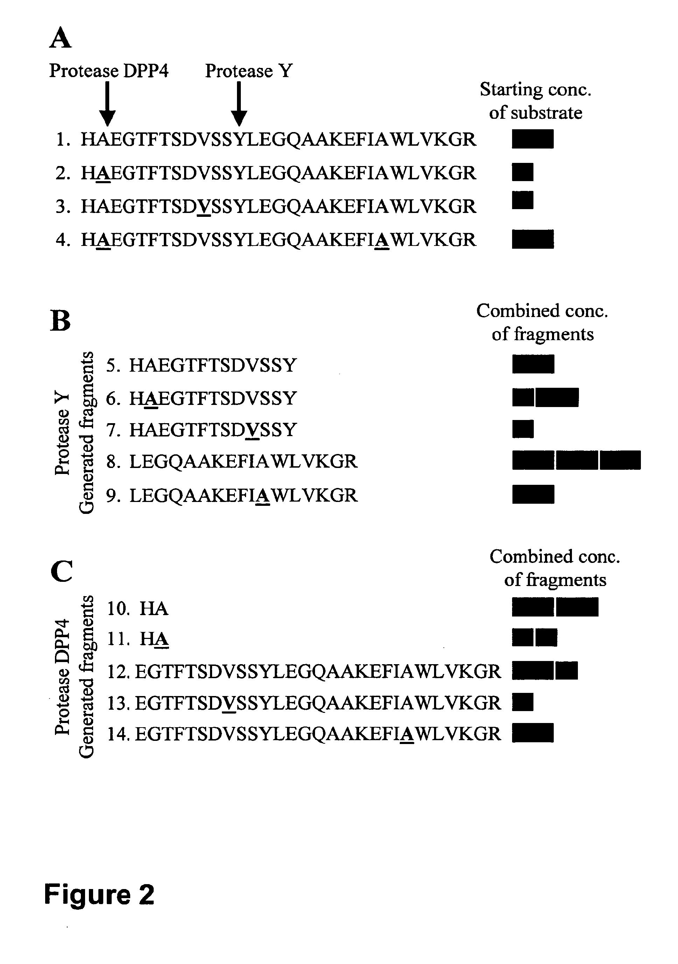

Figure 2 schematically shows the principle of using a set of 4 different, hypothetical variants of a given substrate, wherein the variants have the same amino acid sequence, but differ in individual amino acid residues which are either the "normal" amino acid residues or which are isotope-labeled amino acid residues. Panel A shows four given substrate variants (1. to 4.) all representing the human glucagon-like peptide 1 (GLP-1) amino acid sequence in single letter code. The labeled amino acid residues are in bold, underlined amino acid residues and two proteolytic cleavage sites are indicated by arrows (dipeptidylpeptidase 4 = DPP4, which is known to cleave GLP-1 between amino acid residues 2 and 3 and a second, hypothetical protease termed "Protease Y"). At the right side of panel A are indicated by horizontal bars the hypothetical, relative starting concentrations of the individual variants of GLP-1. Panel B shows the five different hypothetical proteolytic fragments (5. to 9.) which would originate from the four variants of GLP-1 of panel A due to proteolytic activity of the hypothetical protease "Protease Y". At the right side of panel B is schematically indicated the hypothetical relative concentration of these five proteolytic fragments of GLP-1, which for fragments 5, 7 and 9 would originate from a single variant of GLP-1, for fragment 6 would originate from two different GLP-1 variants (the portion of these GLP-1 variants representing fragment 6 is identical for both GLP-1 variants), and for fragments 8 would originate from three different GLP-1 variants. Fragments 5, 6 and 7 all represent the same amino acid sequence but have slightly different molecular weights due to the isotope label and, in addition, fragments 5, 6 and 7 all are present in different relative concentrations. The molecular masses and the relative quantities of the proteolytic fragments 5 to 9 represent a pattern specific for Protease Y. Panel C shows the same kind of hypothetical data as panel B, but for the proteolytic fragments (fragments 10. to 14.) generated from the four GLP-1 variants by the activity of the known protease DPP4.

**Detailed description of the invention**

Protease

The term "protease" according to the invention includes all kinds of enzymes capable to cleave (hydrolyze, cut, digest, etc.) a chemical bond, preferably a peptide bond. According to the invention, peptidases, proteinases, proteolytic enzymes, exopeptidases, endopeptidases, aminopeptidases, carboxypeptidases, etc. are all regarded as proteases.

According to the invention, a first-line protease is a protease which is presumably of major importance for the cleavage of a particular substrate and which preferably has specificity for said substrate. Thus, usually, the specificity for a particular substrate of and high cleavage rate of said substrate by a first-line protease exceeds any other proteolytic activity, e.g. second-line proteolytic acitivity, for said substrate in an organism, the latter proteolytic activity being thus not or only hardly detectable.

According to the invention, a second-line protease for a particular substrate relates to a protease which cleaves said substrate to a minor extent as compared to the cleavage of said substrate by a first-line protease for said substrate. Thus, a second-line protease can be considered as subsidiary protease. A second-line protease cleaves said particular substrate only to a minor extent compared to a first-line protease and the proteolytic activity of the second-line protease is normally overlaid or masked by the proteolytic activity of the first-line protease. However, if the activity of one or more of the first-line proteases is not present, e.g. because one or more of the first-line proteases are inhibited, for example, by use of one or more protease inhibitors for said one or more first-line proteases, the second line protease(s) may cleave a particular substrate in a perceptible extent and may at least in part replace or restore the proteolytic activity of the first-line protease(s) and thereby become "visible". Not every protease cleaving a substrate to a minor extent can be considered as second-line protease, as such protease not necessarily turns out to be a major protease for said substrate once the first-line protease(s) for said substrate is (are) inhibited, especially if proteolysis takes place in vivo in a living organism or in living cells cultured in vitro.

First-line and second-line proteases may only be defined in relation to a particular substrate. Depending on the substrate a protease may be a first-line protease for one substrate while it is a second-line protease for another substrate, and while it is neither a first-line nor a second-line protease for another substrate. It is possible, that there exist for a particular substrate more than one first-line protease and/or more than one second-line protease. The mere sequence and hence the mere presence of a particular protease recognition sequence in a substrate does not necessarily indicate that this substrate in vivo is a substrate for a first-line or a second-line protease. Also the mere presence of particular protease recognition sequences in a substrate does not necessarily indicate that a first-line or second-line protease in vivo cleaves said substrate, even if said protease recognition sequence is a protease recognition sequence for said first-line or second-line protease. It might be, for example, that a protease recognition sequence is sterically not accessible for a proteases and therefore is not cleaved by said protease, or it might be, that the substrate and the protease do not come into contact in vivo, as they are for example located in different compartments within the organism, e.g. blood versus tissue, brain versus heart, etc., or within different compartments of cells, e.g. nucleus versus cytosol, mitochondria versus Golgi apparatus, etc.

In those cases where a first-line protease and a second-line protease cleave a particular substrate exactly at the same position the activities of these proteases are more difficult to distinguish, as both generate the same proteolytic fragments from the substrate. However if time kinetics or dose kinetics (for example of the inhibitor of the first-line protease) are performed, both proteases can be distinguished.

Substrate for a protease and proteolytic fragments thereof

According to the invention, a substrate for a protease is any molecule or composition of molecules which comprises at least one bond susceptible to cleavage by a protease. Cleavage of a substrate by the activity of a protease results in proteolytic fragments. Depending on the number of proteases specific for a particular substrate present in a sample and/or number of cleavage sites on a particular substrate compound for one or more proteases present in a sample, a single substrate compound may upon cleavage result in two or more proteolytic fragments.

With the term "substrate which is naturally present in a biological sample" is meant all substrates, which are present in a biological sample without a human having intentionally added them to the sample. The term "substrate which has been added to a biological sample" relates to the situation where one or more substrates have been directly added to said biological sample or have been administrated to an experimental animal. Such substrates can be administrated to an experimental animal for example by injection of said substrates.

Preferably, a substrate for a protease and proteolytic fragments thereof are peptides and/or proteins. However, a substrate may also comprise non-peptide or non-protein portions. Accordingly, the proteolytic fragments formed by the activity of a protease may also be peptides and/or proteins including non-peptide or non-protein portions.

The terms "sequence" and "substrate comprising a sequence" relates to the composition, in particular amino acid sequence, of a peptide or protein as defined herein and to a substrate as defined herein comprising said sequence, respectively.

Peptides and proteins

The term "peptide" refers to substances comprising two or more, preferably 3 or more, preferably 4 or more, preferably 6 or more, preferably 8 or more, preferably 10 or more, preferably 13 or more, preferably 16 or more, preferably 21 or more and up to preferably 50, in particular 100 or in particular 150 consecutive amino acids joined covalently by peptide bonds. The term "protein" refers large peptides, preferably to peptides with at least 151 amino acid residues, but in general the terms "peptides" and "proteins" are synonyms and are used in this application as synonyms. The terms "peptide" and "protein" according to the invention also include substances containing not only amino acid residues, but also non-amino acid constituents such as sugar or phosphate structures and includes also substances containing only peptide bonds as well as substances containing other bonds, e.g. ester, thioether or disulfide bonds.

A "variant" of a peptide or protein or a "variant of a sequence" as used herein, comprises a peptide or protein or sequence thereof that differs from a peptide or protein from which it is derived, in particular a naturally occurring peptide or protein, in one or more substitutions, deletions, additions, insertions and/or modifications of amino acids. Such variants may be those that exist naturally, or those in which one or more substitutions, deletions, additions, insertions and/or modifications have been introduced. The terms "variant" and "derivative" are used as synonyms herein.

Peptide or protein variants according to the invention include those exhibiting at least about 70%, 75%, 80%, 85%, 90%, 91%, 92%, 93%, 94%, 95%, 96%, 97%, 98%, or 99% or more identity to the peptide or protein sequences from which they are derived.

The peptides or proteins described herein may also comprise any natural and non-natural modifications, in particular chemical or physical modifications. The term "natural modification" relates to any modification found in peptides or proteins in nature such as posttranslational modifications, modifications due to exposure to light, oxygen, exposure to acid or alkali solutions, etc. The term "non-natural modification" relates to non-naturally occurring modifications as found, for example, in peptidomimetics.

Modifications of peptides or proteins described herein may comprise modifications due to posttranslational modifications, chemical modifications, enzymatical modifications and modifications due to other mechanisms. Examples of possible modifications include but are not limited to: glycosylation, phosphorylation, sulphatation, pyroglutamate modification, cystein-disulfide bridges, methylation, acetylation, acylation, farnesylation, formylation, geranylgeranylation, biotinylation, stearoylation, palmitylation, lipolyation, C-mannosylation, myristoylation, amidation, deamidation, methylation, demethylation, carboxylation, hydroxylation, iodination, oxidation, pegylation, prenylation, ADP-ribosylation, addition of lipids, of phosphatidylinositol, of glycosylphosphatidylinositol (GPI)-anchor, of pyridoxal phosphate, modification of cysteine residues resulting in carboxyamidomethylcysteine, resulting in carboxymethylcysteine, or resulting in pyridylethylcysteine, modification of lysine residues resulting in liponic acid, modification of glutamic acid resulting in pyroglutamic acid, etc.

Modifications of peptides or proteins described herein may comprise unusual amino acids, chemically or enzymatically modified amino acids etc. including, but not limited to: alpha amino butyric acid, beta amino butyric acid, beta amino iso-butyric acid, beta alanine, gamma butyric acid, alpha amino adipic acid, 4-amino benzoic acid, amino ethyl cysteine, alpha amino penicillanic acid, allysine, 4-carboxy glutamic acid, cystathionine, carboxy glutamic acid, carboxy amido methyl cysteine, carboxy methyl cysteine, cysteine acid, citrulline, dehydroalanine, di-amino butyric acid, dehydro amino-2-butyric acid, ethionine, glycine-proline di-peptide, 4-hydroxyproline, hydroxylysine, hydroxyproline, homoserine, homo cysteine, histamine, iso-valine, lysinoalanine, lanthionine, norvaline, norleucine, ornithine, 2-pipiridine-carboxylic acid, pyroglutamic acid, pyrrolysine, proline-hydroxy proline di-peptide, sarcosine, 4-selenocysteine, syndesine, thioproline, etc. Further examples can be found in databases such as the "Delta Mass" database searchable at the website of the ABRF, the "Association of Biomolecular Resource Facilities":
http://www.abrf.org/index.cfm/dm.home?AvgMass=all.

Preferably the peptides or proteins described herein are modified to facilitate their detection, alter their stability during storage or usage (for example by using peptidomimetic structures), alter their toxicity or bioavailability or biological half-life, etc. (for example by pegylation), and, in particular, alter their susceptibility towards proteolytic cleavage.

Peptidomimetics

Protease recognition sequences according to the invention may be modified in such a way that the substrate is no longer proteolytically cleaved, or in such a way, that the rate of proteolysis is significantly lowered. This may result in increased half-life of the substrate.

For this purpose substrates can be prepared or synthesized to represent partially or completely peptidomimetics. Furthermore, it can be determined according to the invention whether a peptidomimetic is proteolytically cleaved and the rate of cleavage can be determined. Preferably those "peptidomimetics" or "mimetics of peptides" are used that modify substrates in a way resulting in increased resistance to proteolysis. Peptidomimetics can replace some or all peptide bonds by other kinds of covalent bonds which can connect amino acids. Peptidomimetics among others can comprise modifications of amino acid residues such as alpha-C alkylation, alpha-N alkylation, etc, dipeptide analogues (e.g. two amino acid side chains which are connected by covalent bonds) or modifications of the peptide backbone, especially change from L- to D-amino acid residues, inverse N- to C-sequence, amide bond isosteres, peptoides (e.g. oligomers ofN-substituted glycines, PNAS, 1992, 89:9367), retro-inverso-peptidomimetica (Acc Chem Res, 1993, 26: 266), oligocarbamates (peptide bond replaced by carbamate structure; Science, 1993, 261:1303), oligopyrrolinones (J Am Chem Soc, 1992,114:10672), vinylopeptides (J Am Chem Soc, 1992, 114:6570), etc. Further examples of peptidomimetics suitable for constructing more stable substrates are spiegelmers® (NOXXON Parma AG, Berlin, Germany) or substrates comprising beta amino acids.

Peptide or amino acid sequences furthermore can be protected from proteolytic cleavage by use of amino acid analogues not found or not regularly found in nature. An example for such amino acid analogs are D-amino acids, which are resistant to most proteases. Further examples of amino acid analogues not found in nature, which for example can be used in FMOC-peptide synthesis among others are: Fmoc-2-aminobicyclo[2.2.1]heptane-2-carboxylic acid, Fmoc-3-endo-aminobicyclo[2.2.1]heptane-2-endo-carboxylic acid, Fmoc-3-exo-aminobicyclo[2.2.1]heptane-2-exo-carboxylic acid, Fmoc-3-endo-amino-bicyclo[2.2.1]hept-5-ene-2-endo-carboxylic acid, Fmoc-3-exo-amino-bicyclo[2.2.1]hept-5-ene-2-exo-carboxylic acid, Fmoc-cis-2-amino-1-cyclohexanecarboxylic acid, Fmoc-trans-2-amino-1-cyclohexanecarboxylic acid, Fmoc-1-amino-1-cyclopentanecarboxylic acid, Fmoc-cis-2-amino-1-cyclopentanecarboxylic acid, Fmoc-1-amino-1-cyclopropanecarboxylic acid, Fmoc-D-2-amino-4-(ethylthio)butyric acid, Fmoc-L-2-amino-4-(ethylthio)butyric acid, Fmoc-L-buthionine, Fmoc-S-methyl-L-Cysteine. These analogues are available for example from "The Peptide Laboratory", Benicia, CA, USA.

Labeled substrates

The invention envisions the use of substrates, which may comprise at least one label. The term "label", as used herein, refers to any moiety that functions to: (i) provide a detectable signal; (ii) interact with a second label to modify the detectable signal provided by the first or second label, e.g. FRET (Fluorescence Resonance Energy Transfer); (iii) affect mobility, e.g. electrophoretic mobility, by charge, hydrophobicity, shape, or other physical parameters, or (iv) provide a capture moiety, e.g., affinity, antibody/antigen, or ionic complexation.

The substrates used according to the invention may be labeled to facilitate their detection, to technically improve the detection of the substrates and proteolytic fragments thereof, to reduce the work-load as only the labeled substrates and proteolytic fragments thereof have to be identified, to increase the specificity and/or the sensitivity of the assay, etc.

Suitable as label are structures, such as fluorescent labels, luminescent labels, chromophore labels, radioisotopic labels, isotopic labels, preferably stable isotopic labels, isobaric labels, enzyme labels, particle labels, in particular metal particle labels, magnetic particle labels, polymer particle labels, small organic molecules such as biotin, ligands of receptors or binding molecules such as cell adhesion proteins or lectins, label-sequences comprising nucleic acids and/or amino acid residues which can be detected by use of binding agents, etc.

Label sequences can be bound by binding agents such as antibodies, receptors, PCR-primers, Northern blot probes, etc. Label sequences can be amino acid sequences or nucleic acid sequences or combinations thereof or molecules mimicking the structure of amino acid or nucleic acid sequences (mimetics).

For example, isotopes with molecular weights different to their natural molecular weight such as oxygen-18, nitrogen-15, deuterium, tritium, carbon-14, sulphur-35, phosphorus-32 or phosphorus-33 etc. can be used to prepare isotope-labeled substrates. The isotopes can be incorporated directly into the substrate by using isotope-labeled amino acids for synthesis of the substrate. Furthermore, these isotopes can be incorporated into substrates by growing cells or other organisms expressing said substrate in the presence of isotope-labeled metabolites and isolating the labeled substrate from these organisms, cells or cell culture supernatants. The isotopes can also be incorporated into substrates by other methods such as in vitro translation using isotope-labeled metabolites. Isotope-labeled metabolites can be amino acids, carbohydrates, fatty acids, inorganic salts such as phosphate, sulphate, etc. or other isotope-labeled substances. These isotope-labeled substrates are especially suitable for measuring methods using mass spectrometric methods.

Another kind of label especially suitable for mass spectrometric detection methods are isobaric labels. Different kinds of such labels have the same molecular weight, as long as these labels are intact and have not been fragmented in a mass spectrometer. This has the advantage, that mixtures of molecules labeled with different isobar labels can more conveniently be analyzed using mass spectrometric methods, as only a limited number of mass differences is present prior to analysis of the sample. Fragmentation of the isobar labels is done to determine the identity of the label, thereby identifying the identity of the molecule labeled with the isobar label. Preferably these labels are constructed in a way that upon fragmentation of the label in a mass spectrometer the mass spectrometric fragments generated have masses which usually are not obtained by fragmentation of peptides or proteins. Examples of such masses are masses between 114 and 120 Da. Generally isobaric labels should be selected, which generate masses, which are not generated in the same kind of samples, analyzed by the same mass spectrometric method in the absence of said isobaric labels.

In an alternative to directly using labelled amino acids in peptide synthesis, peptides and proteins generally can be labelled using amine-reactive reagents such as isothiocyanates, succinimidyl esters and carboxylic acids, sulfonyl chlorides, aldehydes, arylating reagents, etc. or thiol-reactive reagents such as iodoacetamides, maleimides, alkylhalides and arylating agents. Furthermore alcohols such as those present in serine, threonine or in carbohydrate moieties can be labelled by oxidizing with periodate to yield aldehydes that can be subsequently modified with a variety of amine or hydrazine derivatives. Alcohols from tyrosine sometimes can be reacted with sulfonyl chlorides or iodoacetamides. Alcohols in carbohydrate moieties furthermore can be labelled by reaction with dichlorotriazines. N-methylisatoic anhydride will convert ribonucleotides and other carbohydrate moieties to fluorescent esters with excitation/emission maxima of about 350/466 nm.

Examples of fluorescent labels are fluorophores such as Alexa Fluor® 350, 405, 430, 488, 500, 514, 532, 546, 555, 568, 594, 610, 633, 635, 647, 660, 680, 700 and Alexa Fluor® 750, Aminomethylcoumarin (AMCA), Methoxy coumarin acetic acid, Bimane, BODIPY 493/503, 530/550, 558/568, 564/570, 576/589, 581/591, 630/650 and BODIPY 650/665, BODIPY FL, BODIPY TR; BODIPY TMR, Cascade Blue dye, Cascade Yellow dye, cyanine dyes such as Cy3, Cy5, Cy5.5, Cy7, Dansyl, Dapoxyl dye, Dialkylaminocoumarin, Eosin, Erythrosin, Fluorescein (FITC), Fluorescein-EX, 2',4',5',7'-Tetrabromosulfonefluorescein, Naphthofluorescein, 2',7'-Dichloro-fluorescein, 4',5'-Dichloro-2',7'-dimethoxy-fluorescein, 6-Carboxy-2',4,4',5',7,7'-hexachlorofluorescein, succinimidyl ester (6-HEX, SE), 5-carboxyfluoresceine (5FAM), 5,6-carboxyfluoresceine (5(6)FAM), hydroxycoumarin, Malachite green, Marina Blue dye, methoxycoumarin, 7-nitro-4-benzofurazanyl (NBD), Oregon Green® 488, Oregon Green® 514, Pacific Blue, Pacific Blue dye, PyMPO, Pyrene, QSY 7 , QSY 9, QSY 21, QSY 35, Rhodamine 6G, Rhodamine Green, Rhodamine Green dye, Rhodamine Red dye, Rhodamine Red, Rhodamine Red-X (RRX), X-rhodamine, Tetramethyl-rhodamine (TMR, TRITC), Lissamine rhodamine B, Texas Red, Texas Red dye, Texas Red-X. Most of these fluorophores are available from Molecular Probes, Eugene, OR, USA, and many are also available as kits intended for protein or peptide labelling.

Furthermore fluorescent proteins or fragments or derivatives thereof such as green fluorescent protein, red fluorescent protein, blue fluorescent protein, renilla fluorescent protein, etc. can be used as labels.

Examples of radioactive isotopes suitable as labels are phosphorous-32 or -33, sulphur-35, iodine-125, tritium, carbon-14, calcium-45, chromium-51 and all other known radioisotopes which can be coupled to nucleic acids and/or peptides or proteins.

It is also possible to use matched pairs of fluorescent labels, which are suitable for fluorescence resonance energy transfer (FRET).

Examples of enzyme labels are horseraddish-peroxidase, alkaline phosphatase, luciferase, galactosidase, etc.

Suitable toxin labels are all kinds of toxins such as microbial toxins, toxic peptides or toxic small organic molecules, synthetic toxins or toxins used for medical purposes.

An example of suitable dye label is digoxigenin.

Examples of particles as labels are particles of various particle diameters, made from various substances, preferably heavy elements such as gold, silver, platinum or other suitable metals or alloys or mixtures thereof. Examples of magnetic particles are preferably made from all kinds of magnetic or magnetizeable materials including iron, preferably iron oxides. Examples of polymer particles are particles made from a polymer such as polyacryl amide, agarose, polypropylene, polystyrene, polytetrafluoroethylene, etc.

Various other labels, such as biotin, chitin, maltose or glutathione etc. as known in the art, can also be used.

Furthermore labels comprising certain additional nucleic acid or amino acid sequences subsequently termed label-sequences can be used. Label-sequences comprising nucleic acid sequences for example can be used for detection of the labeled substance for example by hybridization with a probe specific for the label-sequence or by detection of the label-sequence by polymerase chain reaction, preferably by quantitative polymerase chain reaction, preferably by real-time polymerase chain reaction, etc. Examples of label-sequences comprising amino acid sequences are his-tags, flag-tags, myc-tags or whole proteins or fragments thereof such as antibodies, glutathione S-transferase, streptavidin, chitin-binding protein, maltose binding protein, various lectins, etc. These labels can be detected by use of antibodies or by use of ligands binding to these labels such as protein A, protein G, protein Y, protein A/G, glutathione, biotin, chitin, maltose or other sugars etc. Label-sequences in general can also be used to capture the labelled peptide or protein by a binding agent specific for said label-sequence. Subsequently or during the capture process the labelled peptide or protein can be quantified.

Labels can be present at the N- and/or the C-terminus and/or can be present internal in an amino acid sequence of a substrate and/or can be attached to an amino acid side chain. Preferably two labels are present in a substrate, one at the N- and one at the C-terminus. This enables the detection of all proteolytic fragments of said substrate, if the substrate is cleaved only once.

Proteases present in a biological sample

The term "proteases present in a biological sample" relates to all proteases, regardless if these proteases are naturally present in the sample or if these proteases have been added to the sample, for example by injecting the proteases into an experimental animal or by transfecting the experimental animal or by transfecting in vitro cultured cells or tissues with nucleic acids coding for said proteases, etc.

Examples for proteases are aspartic peptidases, cysteine peptidases, glutamic peptidases, metallo peptidases, serine peptidases, threonine peptidases, amino peptidases, carboxypeptidases, endopeptidases, exopeptidases wherein specific examples are angiotensin converting enzyme (ACE), ACE2, renin, neutral endopeptidase (NEP) (involved for example in hypertension), HIV-1 protease, HIV-2 protease, factor Xa, factor VIIa, factor XIIIa (involved for example in thrombosis), interleukin-1 converting enzyme (ICE) (involved for example in inflammatory diseases), dipeptidyl peptidase 4 (DPP4) (involved for example in type II diabetes), alpha-, beta-, gamma-secretase, Caspase (involved for example in Alzheimer's disease), Cathepsin K (involved for example in osteoporosis), Matrix metalloproteinases (MMPs), proteosome protease (involved for example in cancer), NS3 protease (involved for example in HCV infection).

Proteolytic activity for a given substrate

The term "proteolytic activity for a given substrate present in a biological sample" relates to the proteolytic activity of all kinds of proteases within said biological sample, preferably second-line proteases, which can cleave said substrate into proteolytic fragments. The term "proteolytic activity" in this respect relates to the specificity and efficiency of the cleavage by proteases. It should be noted that the "proteolytic activity" of a protease may depend on the sample, in particular factors such as the concentration of the substrate, the concentration, activity and specificity of the protease, the pH of the sample, the ionic strength of the sample, the presence, the absence, or the concentration of ions such as calcium, sodium, magnesium, potassium etc., and the temperature of the sample.

Furthermore, there may be present different proteases competing for the same substrate or even for the same protease-recognition sequence within the same substrate in a sample. In addition, certain factors of the sample may inhibit or promote the activity of proteases. Factors which might inhibit the activity of proteases for example are factors which covalently or non-covalently bind to and thus, inhibit or inactivate the catalytic domain of the protease, factors which block the substrate binding domain of the protease, or factors which induce a change in the three-dimensional structure of the protease or the substrate, wherein the change of structure inhibits or reduces cleavage of the substrate by the protease. Factors which might promote the activity of proteases for example are factors which are co-factors necessary for efficient substrate binding or cleavage by the protease or factors inducing a change in the three-dimensional structure of the protease or the substrate, wherein the change of structure facilitates proteolysis of the substrate by the protease.

According to the invention, proteolytic activity for a given substrate is present in a biological sample if said substrate if contained in the biological sample is cleaved into one or more fragments by proteases present in said biological sample.

Inhibiting the activity of least one protease

The term "inhibiting the activity of at least one protease" includes the partial or complete inhibition of the activity of at least one protease. Preferably said term relates to an inhibition of the activity of at least 10 %, preferably at least 20 %, preferably at least 30 %, preferably at least 40 %, preferably at least 50 %, preferably at least 60 %, preferably at least 70 %, preferably at least 80 %, preferably at least 90 %, preferably at least 95 %, and in particular completely or almost completely compared to the activity of a protease not being inhibited. The expression "at least" as used in the expression "at least one protease" includes only one entity, up to 2, up to 3, up to 4, up to 5, up to 10, or 10 or more different entities such as different proteases. In cases where the activity of different proteases is inhibited the degree of inhibition of said proteases may be different. In these cases either only one means for inhibiting the activity of proteases, e.g. a single protease inhibitor, or different means, e.g. different protease inhibitors, may be used. The term "different proteases" relates to proteases which have different characteristics, in particular differing in their sequence, structure, protease recognition sequence, substrate specificity, susceptibility to protease inhibitors, requirements regarding proteolytic reaction conditions such as requirement for co-factors, ions, temperature, pH, ionic strength, etc. or combinations thereof. According to the invention, the same protease recognition sequence within a given substrate may be recognized by different proteases and even the same peptide bond within a given substrate may be cleaved by different proteases.

Inhibition of the activity of a protease for example can be achieved by blocking the catalytic moiety of said protease, blocking the binding of said protease to it's substrate, inhibiting the expression, secretion or activation of said protease, increasing the inactivation of said protease (for example by other proteases inactivating said protease), increasing the excretion of the protease (for example by increased renal excretion), removing or blocking essential ions or co-factors needed for the activity of said protease, altering the ionic strength, temperature, pH etc. Usually, the inhibition of a protease results in increased concentrations of substrates for said protease and decreased concentrations of products formed by said protease (proteolytic fragments). A quantitative determination (relative or absolute) of said substrates and/or products can be used to determine the percentage of inhibition. If a protease known to proteolytically cleave a given substrate is not inhibited completely and a different protease which is not inhibited cleaves the same substrate it is possible to distinguish these two identical activities, for example, by kinetic measurements of both proteases, dose-kinetic measurements (different concentrations of inhibitor tested), time-kinetic measurements of the substrate and/or product, or measurements at at least two different time points.

The inhibitor can be administered to the biological sample in any form and by any method such as bolus or as continuous addition, for example by continuous release from a depot, or the inhibitor can be given repeatedly in the same or different doses, preferably 2 doses, preferably 3 doses, preferably 4, doses, preferably 5 doses, preferably up to 10 doses, preferably 10 or more doses, etc. If the inhibitor is given to an organism, preferably to a mammal, the inhibitor can be given by any route such as orally, by subcutaneous, intravenous, intraperitoneal, intralymphatic administration, by direct injection into a tissue or organs such as muscle, fat, skin, liver, kidney, heart, brain, etc., by rectal application, by continuous infusion, by implanting a pump releasing said inhibitor, by application of a matrix continuously releasing said inhibitor for example by diffusion, etc.

It might be, that the inhibitor exhibits different inhibitory efficiencies and/or specificities depending on the species from which the biological sample or the organism/individual originates. Therefore experimental setups might be done using biological samples and/or organisms/individuals originating from more than one species, preferably originating from at least 2 species, preferably of at least 3, at least 4, of at least 5 different species.

Protease known to proteolytically cleave a substrate

According to the invention a protease is known to proteolytically cleave a substrate if it is known, e.g. from general knowledge or earlier experiments, or it can be reasonably expected, e.g. from a comparison with other proteases, that said protease proteolytically cleaves said substrate.

Biological sample

The term "biological sample" according to the invention comprises any material which may be used for testing in the methods according to the invention. The sample may be derived from any source and may include components of different sources. As used herein, a "biological sample" is any sample obtained from or present in a biological system such as viruses, monocellular or multicellular organisms, in vitro cultured cells, in vitro cultured tissues, tissue culture medium or cell culture medium. Monocellular organisms include bacteria, yeasts, in vitro cultured primary cells or cell lines. Multicellular organisms include nematodes such as for example Caenorhabditis elegans (C. elegans), humans, animals, plants, and microorganisms. Preferably the organisms from which the biological samples are obtained are small laboratory animals such as rats, mice, hamsters, guinea pigs, gerbils, rabbits, zebra fish, drosophila flies or larger mammals, preferably pigs, mini pigs, dogs, cats, apes or humans. It is possible to use tissues such as fat, muscle, blood, bone, intestine, pancreas, liver or tumor tissue or to use in vitro cultured primary cells, cell lines or cell culture medium from said tissues as biological samples. The term "biological sample" according to the invention also includes biological samples as defined herein present within a living organism, preferably within their natural environment.

The biological samples can be obtained by methods known in the art such as venous puncture (blood, serum, plasma), directly collecting urine or collecting urine by use of a catheter. Tissue or cells can be obtained by biopsy or collected during surgery, for example blood cells can be sorted for distinct populations of blood cells such as erythrocytes, macrophages, lymphocytes, B-cells, T-cells, etc. using methods known in the art such as density gradient centrifugation or FACS (fluorescence activated cell sorting), etc. Any kind of the above noted materials, or combinations thereof can be used according to the invention. Preferably, the biological sample is a blood, serum, plasma, urine or a tissue sample, preferably obtained from a healthy animal or human or from an animal or human afflicted with a disease or other condition.

According to the invention the biological samples may be samples from organisms subjected to different environmental conditions, or samples from organisms of different health or other conditions or samples from organisms afflicted with or without or with different kinds of diseases, samples from individuals of different genetic or ethnic background etc. Examples of environmental conditions are physical activity, lack of physical activity, psychological stress, mental activity, exposure to UV-, x-ray, electronic- or other irradiation, consumption of food, water, pharmaceutical substances, alcohol, tobacco products, etc. consumption of special diets such as fat-rich, sugar-rich, carbohydrate-rich, protein-rich, fat- and carbohydrate-rich diets etc. All of these environmental conditions may have effects on the concentration, physical location within the body and/or within cells, the activity, the half-life, etc. of proteases, substrates, or co-factors of proteases. All of these environmental factors can have effects as they may result in changes in pH, ionic strength, temperature, viscosity, physical accessibility of substrates for a protease, the presence of natural protease inhibitors, the presence of other proteases competing for a given substrate, or even competing for the same protease recognition site within a given substrate, etc. Examples for health or other conditions are over-weight, under-weight, dehydratation, age, symptoms of age such as muscle resorption and deterioration of joints, female or male gender, fertility-associated conditions such as pregnancy and different stages of menstrual cycle, erectile dysfunction, etc. The samples may originate from individuals having or not having a disease or other condition, from organisms having different diseases or other conditions or from organisms having different stages of diseases or other conditions. Different stages of a disease or other condition include the time points before, during or after the occurrence or first diagnosis of said disease or other condition. Organisms afflicted with a disease can suffer from diseases such as cancer, neurological diseases such as Alzheimer's disease, disorders of the clotting cascade such as deep vein thrombosis, pulmonary embolism, myocardial infarction, stroke and disseminated intravascular coagulation during sepsis, metabolic diseases such as metabolic syndrome, type I and type II diabetes, allergic diseases, autoimmune diseases, inflammatory diseases such as sepsis, infectious diseases such as HIV, hepatitis and malaria, dysregulation of the blood pressure leading to high or low blood pressure, etc. Examples of individuals of different genetic or ethnic background are experimental animals with a genetic knock-out or experimental animals over-expressing a protein or peptide, or individuals of the same species but from different geographic regions such as Caucasian, African and Asian humans, etc.

Fraction of a biological sample

With fraction of a biological sample are meant for example fractions obtained from a biological sample by purifying, fractionating, separating etc. the biological sample with methods such as chromatography, ultrafiltration, precipitation techniques, etc. A fraction might also be a certain subcellular fraction such as cell membranes, cell nuclei, mitochondria, Golgi apparatus, cytosol, etc. of in vitro cultured cells or of cells from an experimental animal or from a human being.

Reference sample / reference value

According to the invention, a "reference sample" may be used to correlate and compare the results obtained from a test sample. The composition of a "reference sample" is usually similar to a test sample but differs from the test sample in certain variables. In preferred embodiments of the methods of the invention a test sample and a reference sample differ in that one or more of the proteases the activity of which is inhibited in the test sample are not inhibited in the reference sample. The terms "reference sample" and "corresponding sample" are used interchangeably herein.

The reference value can be determined empirically by measuring a sufficiently large number of samples. Preferably the reference value is determined by measuring at least 2, preferably at least 3, preferably at least 5, preferably at least 8, preferably at least 12, preferably at least 20, preferably at least 30, preferably at least 50, or preferably at least 100 samples.

Analysing a substrate and/or proteolytic fragments thereof

The term "analysing a substrate and/or proteolytic fragments thereof" includes all methods of measuring, collecting and analyzing data about said substrate and/or proteolytic fragments thereof such as their relative or absolute concentration, their appearance or disappearance, their chromatographic retention times, their physicochemical properties such as hydrophobicity, isoelectric point, etc.

The term "analysing a substrate and/or proteolytic fragments thereof" includes, in particular, the relative or absolute quantification of compounds which may serve as substrates for one or more proteases and/or the proteolytic fragments thereof resulting from the activity of one or more proteases.

The term "analyzing a substrate and/or proteolytic fragments thereof" according to the invention does not necessarily mean that only a particular substrate and/or the proteolytic fragments thereof are involved in analysis. Rather said term also envisions the analysis of more than one substrate for a particular protease and/or the proteolytic fragments thereof and the analysis of also other components of a biological sample or fraction thereof. In particular, said term also includes an analysis of all peptides and/or proteins of a biological sample or fraction thereof which are technically measurable with the methods used. Furthermore, in the case of an analysis of proteolytic fragments said term does not require that all proteolytic fragments of a particular substrate or of particular substrates are to be analysed. Rather, the term "analyzing a substrate and/or proteolytic fragments thereof" also refers to an analysis of only a fraction of the proteolytic fragments, in particular 1, 2, up to 4, up to 6, up to 8 or up to 10 proteolytic fragments of a particular substrate or of particular substrates.

In the methods of the invention, however, it is not necessary to identify which compounds, in particular peptides and/or proteins, in a biological sample or fraction thereof can serve as substrates for the proteases in the biological sample and/or determine their composition and structure. According to the present invention by determining patterns of the components of a sample, in particular peptide and/or protein patterns preferably comprising a plurality of peptides and/or proteins it is possible to identify the activity and/or specificity of one or more proteases present within the sample. Thus, the methods of the present invention may solely rely on the determination of differences and/or changes in the partial or entire pattern of the components, in particular one or more substrates and/or proteolytic fragments thereof, preferably the peptides and/or proteins in a sample rather than on the identification of the composition of the components, one or more substrates and/or proteolytic fragments thereof, and peptides and/or proteins, respectively, in a sample. However, the methods of the present invention do not exclude embodiments wherein the composition of the components, one or more substrates and/or proteolytic fragments thereof and peptides and/or proteins, respectively, is identified.

In this respect, the terms "identification of the composition of components", "identification of the composition of one or more substrates and/or proteolytic fragments thereof" and "identification of the composition of peptides and/or proteins" relates to the full or partial identification of the chemical structure of said components, one or more substrates and/or proteolytic fragments thereof and peptides and/or proteins, respectively, and, in particular, the full or partial identification of the sequence of one or more peptides and/or proteins. The amount of substrate determined in a biological sample will decrease and the amount of proteolytic fragments of the substrate (= products) will increase in the sample with increasing amount and/or activity of a protease specific for said substrate in the sample. Thus, a small amount of substrate and/or high amount of proteolytic fragments of the substrate determined in a sample is indicative for a high amount and/or activity of protease specific for said substrate in the biological sample. A large amount of substrate and/or small amount of proteolytic fragments of the substrate determined in a sample is indicative for a small amount and/or low activity of a protease specific for said substrate in the biological sample.

The expression "analysing a substrate and/or proteolytic fragments thereof" according to the invention also includes situations wherein no proteolytic fragments of a particular substrate are detected or the amount of said proteolytic fragments is below the detection limit which situations may be indicative for the fact that no protease specific for said particular substrate is present in the sample, said protease is substantially or completely inactive or the activity of said protease is substantially or completely inhibited.

Furthermore, the expression "analysing a substrate and/or proteolytic fragments thereof" also includes situations wherein a substrate is not detectable either because it is not present in the sample or because its concentration is below the detection limit of the test system used which situations may be indicative for the fact that protease specific for said particular substrate is present in the sample in high amounts or said protease has high activity.

On the basis of the amount of substrate or the proteolytic fragments thereof determined in a biological sample or fraction thereof it is possible in the methods of the invention to draw conclusions about the activity and/or specificity of proteases present in the biological sample. In this respect, the activity of a protease may be calculated as a relative or an absolute activity, depending on the experimental setup.

The term "analysing substrate and/or proteolytic fragments thereof" also includes a combination and/or comparison of data obtained from at least two samples, and/or a comparison of data obtained from at least one biological sample with data obtained from at least one reference sample. The methods of the invention can be applied to more than one sample, in particular to create a mean. The data of the biological samples and the reference samples can be determined by measuring a sufficiently large number of samples. Preferably a mean value is determined by measuring at least 2, preferably at least 3, preferably at least 5, preferably at least 8, preferably at least 12, preferably at least 20, preferably at least 30, preferably at least 50, or preferably at least 100 samples.

Reference samples and biological samples can be measured in parallel and then compared to each other. Alternatively reference samples and biological samples can be measured separately and can then be compared, once both measurements are done. The order in which the reference sample and the biological sample are measured can be changed, e.g. the reference sample can be measured first and then the biological sample and vice versa.

An experimental setup to determine whether proteolytic activity for a particular substrate is present in a biological sample or a fraction thereof may be conducted, for example, by measuring the amount or concentration of said substrate and/or proteolytic fragments thereof. This amount or concentration (relative or absolute) of said substrate and/or proteolytic fragments thereof may be compared with a reference value obtained from a reference sample. Thus, it is possible to e.g. determine qualitatively and/or quantitatively the substrate and/or proteolytic fragments originating from said substrate due to proteolytic activity present in said biological sample. The amount of said substrate and/or proteolytic fragments thereof can be reflected by a signal intensity in a test system, for example a mass spectrometric signal intensity, an absorption value, fluorescence value, radioactive counts, luminescence value, etc. of an ELISA (enzyme-linked immuno-sorbet assay), a RIA (radio immuno assay), a protein- or DNA-chip assay, a FACS (fluorescence activated cell sorting) analysis, an immune precipitation, a quantitative PCR (polymerase chain reaction) or RT-PCR (reverse transcriptase-PCR) reaction, or densitometric signal intensity of an exposed film (for example from a Western blot, a Northern blot, or an in situ hybridization), etc.

Generally all methods suitable to detect, characterize and analyze protease substrates and the proteolytic products thereof, in particular peptides and proteins, can be used in the methods of the invention. These methods can be used to determine the presence, absence and/or quantity of said substrate and/or of the proteolytic fragments thereof.

Thus these methods can be used indirectly to identify proteases, for example, by identifying proteolytic fragments generated from a particular substrate due to the activity of said proteases. Preferably mass spectrometric methods, protein chip assays, immunological and molecular biology methods can be used for this purpose.

Preferably peptides of a molecular weight up to 100 kDa, preferably up to 80 kDa, up to 70 kDa, up to 60 kDa, up to 50 kDa, up to 40 kDa, up to 30 kDa, up to 20 kDa, up to 10 kDa and preferably up to 5 kDa are determined.

In certain embodiments of the methods of the invention, high-molecular weight peptides or proteins and other substrates such as biopolymers which might interfere with the measurement are removed from the biological sample prior to measurement. Suitable methods for this among others are native or denaturing gel electrophoresis, 2-D gel electrophoresis, liquid chromatography such as anion or cation exchange chromatography, metal chelate affinity chromatography, affinity chromatography, reversed phase chromatography, gas chromatography, capillary chromatography, thin layer chromatography, mass spectrometry, precipitation techniques such as trichloric acetic acid or trifluoric acid or ethanol precipitation, immune precipitation, liquid phase extraction techniques, filtration and other molecular size discriminating techniques such as gel filtration, ultrafiltration using membranes with for example size exclusion limits of 3, 5, 10, 30 or 50 kilo Dalton, etc.

The biological sample and/or reference sample tested may be divided into fractions for analysis and the fractions may be analyzed with different measuring arrangements and methods.

Suitable mass spectrometric methods among others are matrix assisted laser desorption ionisation (MALDI), continuous or pulsed electrospray ionization (ESI), SELDI mass spectrometry, FAB mass spectrometry and MS/MS mass spectrometry and related methods such as ionspray or thermospray or massive cluster impact (MCI). The ion sources can be matched with detection formats including linear or non-linear reflection time-of-flight (TOF), single or multiple quadrupole, single or multiple magnetic sector, fourier transform ion cyclotron resonance (FTICR), ion trap, and combinations thereof, e.g. ion-trap/time-of-flight. For ionisation, numerous matrix/wavelength combinations (MALDI) or solvent combinations (ESI) can be used. Other mass spectrometric methods suitable are for example fast atom bombardment (FAB) mass spectrometry, Surface Enhanced Laser Desorption/Ionisation (SELDI) mass spectrometry, isotope coded affinity tag (ICAT) mass spectrometry, or affinity mass spectrometric methods.

Furthermore suitable immunologic methods among others are enzyme linked immuno assays (ELISA), sandwich, direct, indirect, or competitive ELISA assays, enzyme-linked immunospot assays (ELISPOT), radio immuno assays (RIA), flow cytometry assays (FACS = fluorescence activated cell sorting), immunohistochemistry, Western blot, fluorescence resonance energy transfer (FRET) assays, protein-chip assays using for example antibodies, antibody fragments, receptors, ligands, or other binding agents specific for peptides, proteins, substrates and fragments thereof.

Further methods such as nuclear magnetic resonance (NMR), fluorometry, colorimetry, radiometry, luminometry, or other spectrometric methods, liquid chromatography, capillary chromatography, thin-layer chromatography, plasmon resonance (BIACORE), one- or two-dimensional gel electrophoresis, etc. can be used to detect substrates and proteolytic fragments thereof, in particular peptides and proteins according to the invention.

Peptide and/or protein pattern

The methods of the invention are preferably performed by generating protein and/or peptide patterns which patterns comprise at least two distinct signals and preferably comparing protein and/or peptide patterns generated. The protein and/or peptide patterns preferably include signals of at least one substrate of at least one protease and/or proteolytic fragments thereof. A comparison of protein and/or peptide patterns may reflect different amounts of substrates and/or proteolytic fragments and thus, be indicative for different activities of at least one protease in a sample.

Such protein and/or peptide pattern preferably comprises two or more, preferably 4 or more, more preferably 6 or more, 8 or more, 10 or more, 15 or more, even more preferably 20 or more, 30 or more, 40 or more, 50 or more, 60 or more, 70 or more, 80 or more, 90 or more, 100 or more, 120 or more, 140 or more, 160 or more, 180 or more, 200 or more, 250 or more, 300 or more, 400 or more, 500 or more signals for peptides and/or proteins. The upper limit of said signals is not particularly restricted as long as the number of said signals and thus, the number of peptides and/or proteins determined does not exceed the resolution limit of the assay system and the available computing power to calculate the correlations. An upper limit may be for example not more than 20, 40, 60, 80, 100, 150, 200, 250, 300, 400, 450, 500, 1000, 2000, 5000 or 10000 signals depending on the assay system used in the methods of the invention. In certain embodiments of the methods of the invention, only a part or fraction of the peptides and/or proteins resulting from proteolytic cleavage is determined which preferably results in less signals in a protein and/or peptide pattern. In these embodiments the peptides and/or proteins to be determined may be subjected to separation means such as chromatographic separations before being determined. For evaluation of such patterns preferably at least one of the following mathematical methods to calculate correlations is used: "Pearson Product-Moment Correlation", "Spearman's Rank-Order Correlation", "Kendall's Tau", "Kendall's Coefficient of Concordance", "Goodman and Kruskal's Gamma", "Manhattan distance", "Euclidean distance" and "Minimal Spanning Tree Diameter". Most preferably "Spearman's Rank-Order Correlation" is used.

Correlations can be calculated for two or more proteolytic fragments or between one or more proteolytic fragments and additional meta data, which might be available. Suitable meta data are protease activity, concentrations of compounds or compositions optionally used to treat an individual or organism from which the biological sample or the reference sample originated, which compounds or compositions preferably comprise protease inhibitors. Further suitable meta data among others are absence or presence of a disease or other conditions, state of the disease, other clinical parameters such as blood pressure, body temperature, medication for disease treatment, age, sex, exposure to environmental conditions, etc.

Identification of proteases

The term "determination of at least one full or partial sequence of at least one of said proteolytic fragments" relates to the full or partial identification of the composition, in particular sequence, of one or more proteolytic fragments generated, in particular the N-and/or C-terminal amino acid sequences thereof. Preferably the amino acid sequence identified comprises at least 2, preferably at least 3, preferably at least 4, preferably at least 5, preferably at least 6, preferably at least 8, preferably at least 10 amino acid residues. In one embodiment, the amino acid sequence identified comprises 1 amino acid residue. The amino acid sequence of a proteolytic fragment may be determined completely, however it is not necessary to determine the complete sequence. Instead it is sufficient to determine only part of the sequence (partial sequence).

The sequence information of the proteolytic fragment, optionally combined with the known sequence of the complete substrate from which the proteolytic fragment originated (known for example from protein data bases such as SwissProt; www.swissprot.com, Swiss Institute of Bioinformatic, Basel, Switzerland) can be used to determine which amino acid residues represent the protease recognition and/or cleavage sequence. The protease recognition sequence usually includes amino acid residues adjacent to the peptide bond which is cleaved by the protease and in addition may include additional amino acid residues within a distinct distance from the position cleaved by the protease, which additional amino acid residues for example may be necessary for interaction of the substrate and the protease. The composition includes the amino acid sequence as well as postranslational modifications such as glycosylation, phosphorylation, chemical modifications, such as oxidized amino acid side chains, acetylation, amidation, etc.

Knowledge about the protease recognition and/or cleavage sequence may allow to draw conclusions about the protease or group of proteases which produced the proteolytic fragment(s).

The following describes preferred non-limiting embodiments of the invention.

Modes for carrying out the invention / Use of GLP-1 as substrate:

I. Non-labeled GLP-1 as substrate

On the basis of the amino acid sequence of a substrate with the number of amino acid residues being n all theoretically possible proteolytic fragments of said substrate can be calculated. Given that the first-line protease is completely inhibited and there is only one second-line protease, which second-line protease cleaves said given substrate only at one position, the maximal number of all theoretically possible proteolytic fragments can be calculated with the following formula:

$$(n-1) * 2 = \text{number of all theoretically possible proteolytic fragments}$$

If the number n of amino acid residues in a substrate for example is 30 (mature GLP-1 for example), then the number of all theoretically possible proteolytic fragments is (30-1) * 2 = 58. If there is more than one second-line protease and/or if the first-line protease is not inhibited completely and/or a first- and/or second-line protease cleaves the given substrate more than once, then there are more theoretically possible proteolytic fragments, and the number of proteolytic fragments has to be calculated with other equations.

For each of these 58 proteolytic fragments the molecular mass can be calculated for example by using the software tool GPMAW, version 5, Lighthouse Data, Odense, Denmark or using software like Microsoft Excel in combination with lists of the masses of amino acid residues. The relative small number of proteolytic fragments of a given substrate can easily be monitored by their mass using mass spectrometry, even from complex samples.

If the biological sample is separated into fractions prior to analysis, for example prior to analysis with mass spectrometry, it is possible to predict the chromatographic elution behavior (= fraction number) based on the known sequence of the proteolytic fragments (EP 1553515 A1). The calculated molecular mass and the predicted fraction number of all theoretically possible proteolytic fragments of a given substrate can be combined to an interest list (see Table 1). This interest list can be used as a coordinate list for a peptide map and can be projected onto a peptide map (see Figure 1). Subsequently only the mass spectrometric signals of the coordinates present in the interest list are further analyzed.

Table 1: All theoretically possible proteolytic fragments of GLP-1 if only a single proteolytic cleavage takes place, their calculated average mass with the mass of 1 proton added (due to mass spectrometrc method), their predicted fraction during reverse phase chromatography, and predictions, whether these proteolytic fragments might originate due to proteolytic activity of a distinct protease using the online tool "PeptideCutter" from the Swiss Institute of Bioinformatics, Geneva, Switzerland.

| GLP-1 Sequence | Average mass + 1 Proton (as measured in mass spectrometry) | Predicted fraction | Predicted protease generating said proteolytic fragment (PeptideCutter) |
|---|---|---|---|
| 1-30 | 3299.7 | 60 | |
| 1-29 | 3162.5 | 63 | |
| 1-28 | 3091.4 | 63 | |
| 1-27 | 2962.3 | 62 | |
| 1-26 | 2905.3 | 63 | |
| 1-25 | 2804.2 | 63 | |
| 1-24 | 2657 | 62 | |
| 1-23 | 2555.9 | 62 | |
| 1-22 | 2468.8 | 62 | |
| 1-21 | 2353.7 | 62 | |
| 1-20 | 2254.6 | 62 | Lys C, Trypsin |
| 1-19 | 2167.5 | 62 | |
| 1-18 | 2080.4 | 62 | |
| 1-17 | 1917.3 | 58 | |
| 1-16 | 1804.1 | 53 | |
| 1-15 | 1675 | 53 | |
| 1-14 | 1617.9 | 53 | |
| 1-13 | 1489.8 | 55 | |
| 1-12 | 1418.7 | 55 | |
| 1-11 | 1347.6 | 55 | |
| 1-10 | 1219.5 | 57 | |
| 1-9 | 1090.4 | 56 | |
| 1-8 | 943.2 | 55 | Asp-N |
| 1-7 | 830 | 53 | |
| 1-6 | 758.9 | 53 | Chymotrypsin |
| 1-5 | 572.7 | 48 | Pepsin |
| 1-4 | 459.6 | 43 | |
| 1-3 | 360.4 | 43 | Glutamyl endopeptidase, Staphylococcal peptidase I |
| 1-2 | 232.3 | 44 | Proteinase K |
| 1 | 175.2 | 45 | |
| 2-30 | 3143.5 | 60 | |
| 3-30 | 3086.4 | 61 | |
| 4-30 | 2958.2 | 62 | |
| 5-30 | 2859.1 | 62 | |
| 6-30 | 2746 | 57 | |
| 7-30 | 2559.7 | 52 | |
| 8-30 | 2488.7 | 52 | |
| 9-30 | 2375.5 | 50 | Asp-N |

(continued)

| GLP-1 Sequence | Average mass + 1 Proton (as measured in mass spectrometry) | Predicted fraction | Predicted protease generating said proteolytic fragment (PeptideCutter) |
|---|---|---|---|
| 10-30 | 2228.3 | 49 | |
| 11-30 | 2099.2 | 48 | |
| 12-30 | 1971 | 50 | |
| 13-30 | 1900 | 50 | |
| 14-30 | 1828.9 | 50 | |
| 15-30 | 1700.8 | 52 | |
| 16-30 | 1643.7 | 52 | |
| 17-30 | 1514.6 | 52 | |
| 18-30 | 1401.4 | 47 | |
| 19-30 | 1238.2 | 43 | |
| 20-30 | 1151.2 | 43 | |
| 21-30 | 1064.1 | 43 | |
| 22-30 | 965 | 43 | Glutamyl endopeptidase, Staphylococcal peptidase I |
| 23-30 | 849.9 | 43 | |
| 24-30 | 762.8 | 43 | |
| 25-30 | 661.7 | 43 | |
| 26-30 | 514.5 | 42 | |
| 27-30 | 413.4 | 42 | Pepsin, Thermolysin |
| 28-30 | 356.4 | 43 | Proteinase K |
| 29-30 | 227.2 | 42 | Lys C, Trypsin |

Alternatively to calculation of all theoretically possible proteolytic fragments of a given substrate, it is possible to select certain proteases, which potentially are present, or expected to be present in a biological sample or which for other reasons might cleave the substrate. Using bioinformatic tools known in the art and described exemplarily herein, it is possible to predict, considering the selected proteases and the given substrate, the proteolytic fragments generated from said given substrate, by proteolytic activity of said selected proteases (row 4 in table 1).

The identification of pre-calculated proteolytic fragments of said substrate out of a complex biological sample can be performed by different means using mass spectrometric and chromatographic information. In the simplest form the mass spectra of all fractions are searched for the proteolytic fragments using the pre-calculated mass and the pre-calculated chromatographic fraction coordinates (interest list) for the occurrence of matching peaks. If scanning mass spectrometers such a quadrupole mass analyzer or ion traps are used, this comprises also the methods of single ion monitoring scanning only the signal intensities at interest list masses. Alternatively, giving a higher specificity, if biological samples with titration series of said given substrate are analyzed, the mass spectrometric signal intensities of the interest list can be correlated against the amount of said substrate added to said biological sample. This allows filtering out the proteolytic fragments in the interest list, which originate from said biological sample. Alternatively, or additionally, the specificity of identifications of proteolytic fragments can be further increased by additional information from mass spectrometric fragment ions in MS/MS experiments. Principally, a combination of two mass analyzers is used in MS/MS experiments. After ionizing the peptides of the fraction of interest via an appropriate ionization source, the first mass analyzer selects a mass over charge ratio of choice (precursor m/z), followed by a specialized compartment of the instrument, typically a collision cell, breaking the precursor molecules into smaller fragments. The second mass analyzer consecutively records the characteristic fragment ion masses. To identify the proteolytic fragments of said substrate all signals at all coordinates or at selected coordinates of the interest list are subjected to MS/MS experiments for example using Electro Spray Ionization (ESI) or Matrix Assisted Laser Desorption Ionization (MALDI) MS/MS instruments. Identification of the proteolytic fragments (precursors) is carried out by the observation of characteristic mass spectrometric fragment ions of the proteolytic fragments. This method also comprises the so called selected or multiple reaction monitoring using scanning mass spectrometers specifically monitoring for single fragment ion masses of selected precursors (transitions). The identification specificity for the proteolytic fragments of said substrate can be further enhanced if series of fragment ions are observed, which are sufficient for an unambiguous identification of the amino acid sequence of the proteolytic fragments by means of a database search. In this case the mass spectrometric fragment ion information is matched against a database containing predicted mass spectra of vast numbers of peptides and proteins using scoring algorithms to judge similarity or identity. To reduce the complexity of this database of predicted mass spectra and to increase the specificity of the identification of the sequence or of part of the sequence of the proteolytic fragments of said given substrate it is possible to compile a small database only containing the predicted mass spectra of all theoretically possible proteolytic fragments of said given substrate. All of the above-described methods can be furthermore combined to improve the identification specificity of the proteolytic fragments of said substrate

## II. Identification of proteolytic fragments using stable isotope pattern

To improve the identification of the proteolytic fragments of a given substrate, it is possible to use a substrate, comprising amino acid residues labeled with isotopes, preferably labeled with stabile isotopes such Leucine $^{15}$N (delta m = 4 Dalton, as compared to Leucine $^{14}$N) or Alanine $^{15}$N (delta m = 7 Dalton) which are chemically and biologically indistinguishable and do not affect protease activity but can be separated by mass spectrometry as a consequence of their characteristic mass shift, as compared to substrates not containing isotope labeled amino acid residues.

At least two isotope variants or the non-modified substrate and at least one isotope variant of said substrate are administered in different concentrations resulting in a mass and signal intensity coded pattern of co-eluting substrates and proteolytic fragments thereof. For example the following hypothetic isotope variants of GLP-1 could be used (bold-type, underlined amino acid residues are labeled with stable isotopes):

HAEGTFTSDVSSYLEGQAAKEFIAWLVKGR non-modified GLP-1
H**A**EGTFTSDVSSYLEGQAAKEFIAWLVKGR Alanine at position 2 is labeled
HAEGTFTSD**V**SSYLEGQAAKEFIAWLVKGR Valine at position 10 is labeled
H**A**EGTFTSDVSSYLEGQAAKEFI**A**WLVKGR Alanine at positions 2 and 24 is labeled

Using this mixture of these 4 different GLP-1 peptides with different GLP-1 variants present in different concentrations, there would be 4 different mass peaks of different signal intensity for the non-cleaved substrate. If all 4 variants of the substrate are cleaved at the same single position there would be generated 8 proteolytic fragments. All pairs of proteolytic fragments would have the same sequence, regardless from which substrate variant they originated, but the molecular weight of the proteolytic fragments of some proteolytic fragments would be identical and the molecular weight of others would be different, due to the presence of isotope-labeled amino acid residues with different than "normal" molecular weights (bold-type, underlined amino acid residues). In addition the quantity of the individual proteolytic fragments depends on the concentrations of the differently labeled substrates administrated from which a distinct proteolytic fragment was generated. This results in a distinct pattern of proteolytic fragments regarding molecular weight and signal intensity (fragment concentration), which facilitates the identification of the cleavage position within said given substrate, especially if mass spectrometry-induced fragmentation of the proteolytically generated fragments of the substrate fails or is insufficient. This principle is depicted in figure 2.

As described in the previous example, the pattern (mass and relative or absolute signal intensity) of all theoretical proteolytic fragments generated due to proteolysis of the non-labeled and/or the isotope-labeled given substrate can be pre-calculated. Subsequently these pattern of signals are searched in the biological sample. Alternatively it is also possible to pre-calculate the pattern if certain distinct proteases are expected to be present in the biological sample. Furthermore it is possible to pre-calculate the mass spectrometric fragment spectra and signal intensities which these mixtures of isotope-labeled given substrates would generate. This reduces the complexity of the database used for comparison and identification of the measured fragment spectra of the proteolytic fragments of the given substrate.

It is important to point out, that there are two kinds of fragments of the given substrate:

- fragments of the given substrates, which are generated due to proteolytic activity present in the sample (proteolytic fragments), and
- fragments generated from the proteolytic fragments, which fragments are generated during the mass spectrometric measurement process by bombarding the proteolytic fragments with molecules of a collusion gas in the mass spectrometer (=mass spectrometric fragments). These mass spectrometric fragments are used to identify the sequence of the proteolytic fragments.

## III. Identification of proteolytic fragments using radioactive isotope pattern

This method is similar to the method described in section II, above, except that the isotope labels of the given substrate consist of amino acid residues labeled with radioactive isotopes such as Leucine $^{14}$C (delta m = 12 Dalton, beta-decay, half-life = 5130 years) or Cysteine $^{35}$S (delta m = 3 Dalton, beta-decay, half-life = 87 days). Different variants of a given substrate in different starting concentrations are used. The theoretical proteolytic fragments, fragment patterns and intensities and the mass spectrometric fragment spectra can be pre-calculated as shown in table 1. The only difference to the method described in section II is, that the biological sample can be fractionated and the individual fractions can be checked for the presence or absence of radioactivity originating from the isotope-labeled, given substrate and from the proteolytic fragments thereof. Only those fractions containing radioactivity may be further analyzed for proteolytic fragments of said given substrate. Detection of radioactive fractions for example can be done by liquid scintillation counting of aliquots of the fractions or by radioactive imaging of small aliquots of the individual fractions for example spotted onto MALDI targets wherein subsequently only the radioactive spots on the MALDI targets are analyzed by mass spectrometry. All of the methods described in sections I, II and III, above, result in improved specificity and/or improved sensitivity. This allows to reduce the amount of a given substrate to be added to the biological sample. This has several advantages such as reducing the risk, that the in vivo amount of second-line protease is limiting and reducing the risk, that the substrate has to be used in non-physiological concentrations to give a significant signal intensity of the proteolytic fragment or reducing cost and experimental time, as less samples have to be measured and less data have to be analyzed using smaller data bases for sequence determination.

Substrate and possible proteolytic fragments resulting therefrom

The methods of the invention are not limited regarding the number of first-line proteases and second-line proteases and number of cleavage sites for each of said proteases in a particular substrate. However, preferably the substrate is cleaved only once by a first-line protease and is cleaved only once by a second-line protease. More preferably the first-line protease is (almost) completely (e.g. more than 95%) inhibited and the second-line protease cleaves the given substrate only once, resulting in only 2 proteolytic fragments. If the first-line protease cleaves the given substrate only once and is not inhibited completely and the second-line protease cleaves the given substrate only once there are already 5 different possible proteolytic fragments generated from the given substrate. The corresponding numbers of proteolytic fragments can be found in table 2. So with increasing numbers of protease recognition sequences there are generated increasing numbers of different proteolytic fragments, all from a single given substrate, resulting in a pattern of such proteolytic fragments, which can be detected according to the invention. Such pattern can be statistically analyzed, as the proteolytic fragments of one cleavage reaction usually should correlate quantitatively with each other and with other proteolytic fragments which originated from the same given substrate. The pattern found in a biological sample and a reference sample can also be compared and statistically analyzed. Such pattern at least comprises two proteolytic fragments of said given substrate. However, as table 2 shows, higher numbers of proteolytic fragments are possible.

Table 2: Maximal numbers of proteolytic fragments generated due to proteolysis of a given substrate, as a consequence of the indicated numbers of second-line proteases / second-line protease recognition sequences and if there is only one first-line protease, which first-line protease cleaves said given substrate only once

| Number of second-line proteases (protease recognition sequences) | 1 | 2 | 3 | 4 | 5 |
|---|---|---|---|---|---|
| max. number of possible proteolytic fragments, if first-line protease is completely inhibited | 2 | 5 | 9 | 14 | 20 |

(continued)

| Number of second-line proteases (protease recognition sequences) | 1 | 2 | 3 | 4 | 5 |
|---|---|---|---|---|---|
| max. number of possible proteolytic fragments, if first-line protease is only partially inhibited | 5 | 9 | 14 | 20 | 27 |

The pattern preferably include signals of a given substrate and of proteolytic fragments thereof, which proteolytic fragments are generated due to the activity of at least one protease. A comparison of patterns of proteolytic fragments may reflect different amounts of first- and second-line protease activity and thus, be indicative for different activities of said proteases in biological and in reference samples. Statistical analysis of these patterns of proteolytic fragments can be used to qualitatively and quantitatively predict the activities of the first- and second-line proteases involved in the production of these patterns.

**Examples**

The following examples are intended to illustrate the invention, however they are not meant to limit the scope of the invention in any way.

Example 1: Sample preparation:

Eight weeks old male Wistar rats (315 to 320 g body weight) are fasted for 1 to 10 h. One group of rats receives a dose of a DPP4 inhibitor which results in maximal inhibition of DPP4. Preferably the DPP4 inhibitor is injected subcutaneously, preferably into the fat tissue of the neck. The control group of rats receives an injection of sodium phosphate (e.g. 50 mM sodium phosphate buffer; pH 7.4, or 0.9 % NaCl solution) as vehicle control. After 1 h all rats receive an injection of a DPP4 substrate, such as GLP-1, preferably by intravenous injection. 60 min., 70 min. and 80 min. after DPP4-inhibitor injection (e.g. 0 min., 10 min. and 20 min. after DPP4-substrate injection), 2.5 ml of blood are drawn from each rat using EDTA (final concentration of 2.5 mg/ml EDTA) or citrate as anticoagulant. If required, rats are anaesthetized using a mixture of Ketamin (9 mg/100 g body weight) and Rompun (0.2 mg/100 g body weight). Additionally, rats may receive a short burst of ether for quick anesthesia. To collect larger volumes of blood, rats can be anaesthetized and the thorax opened immediately and 4 to 6 ml blood can be taken from the right ventricle. Preferably groups of rats are treated with the same experimental scheme, preferably each group contains at least 1, preferably at least 4, preferably at least 8, preferably at least 16 preferably at least 32 animals. It might be, that certain treatment groups of rats, for example the vehicle-control group, contain smaller numbers of animals than other experimental groups. Preferably the blood is drawn retro orbitally. Each blood sample immediately is centrifuged for 10 min. at room temperature at 2000x g. The supernatant (= plasma) is collected and centrifuged for another 15 min. at room temperature at 2500x g. The plasma is collected and stored at -80 °C until further analyzed. If GLP-1 is used as substrate preferably a bolus dose of 1 to 20000 $\mu$g/kg body weight of human GLP-1, preferably 1 to 4000 $\mu$g/kg, preferably 1 to 1000 $\mu$g/kg, preferably 1 to 250 $\mu$g/kg, preferably 1 to 50 $\mu$g/kg, preferably 1 to 10 $\mu$g/kg body weight of human GLP-1 is injected. Preferably human, rat or murine GLP-1 is used. The GLP can be recombinant or can be purified from a natural source. If for example AB 192 (bis(4-acetamidophenyl)-1-(S)-prolylpyrrolidine-2-(R,S)-phosphonate) (J Med Chem, 1999, 42:1041-52) is used as DPP4 inhibitor preferably doses of 0.001 to 100 mg/kg, preferably 0.001 to 10 mg/kg, preferably 0.01 to 10 mg/kg, preferably 0.1 to 10 mg/kg, most preferably doses of 0.32, 1.00 and 3.2 mg/kg body weight (rats) may be used. If other DPP4 inhibitors or other experimental animals or test organisms such as in vitro cultured human, murine, rat, etc. primary cells or cell lines are used, GLP-1 and DPP4-inhibitor concentrations different to those stated might be suitable.

If the given substrate, such as GLP-1 is labeled for subsequent identification of the proteolytic fragments of the given substrate, the dose of the substrate injected into the rats is in the lower end of the concentration range stated.

Preferred labels for a substrate are radioactive isotope labeled or stable non-radioactive isotope labeled substrate. Further preferred embodiments are ICAT-labeled, and fluorescence labeled substrates. Lower substrate concentrations have the advantage, that the experimental in vivo situation is closer to the physiological in vivo situation regarding the ratio of substrate to proteases (first- and second-line proteases) and that there is a lower risk, that the in vivo concentration of the protease is limiting for generation of proteolytic fragments from the given substrate.

Alternatively to in vivo testing it is possible to conduct the above described experiments in vitro, for example by incubation of the DPP4-inhibitor and of GLP-1 in a biological sample such as rat or human plasma, or whole blood. In vitro tests would also allow the determination of proteolytic degradation products of the DPP4 substrate GLP-1 in a human system (human plasma, human whole blood), instead of in experimental animals. Furthermore the use of a human in vitro system may be an advantage, if the DPP4-inhibitor used is more efficiently inhibiting human DPP4, as compared to rat DPP4 or DPP4 of other species.

Example 2: Measurement of DPP4 activity

10 $\mu$l of plasma sample is pipetted in duplicates directly in a 96-well microtiter plate and placed on ice. After all samples and standards (0-0.5 mU/ml purified DPP4 enzyme for example from porcine kidney; Sigma-Aldrich Chemie GmbH, Munich, Germany) are added, the plate is placed on a 37°C heating block and 40 $\mu$l freshly prepared substrate solution (500 $\mu$M G-P-4 nitroanilide diluted in 0.4 mM HEPES buffer) added to each well. The 96-well plate is read immediately using for example a Tecan Genios micro plate reader (Tecan Deutschland GmbH, Crailsheim, Germany) and measurements are taken at 37°C over a period of time of 1h at a wavelength of 405 nm. From the measured values the blank signal (plasma + HEPES buffer) is subtracted and data calculated as Units/liter (U/1). The measurement values of the DPP4 activity are useful for determining the efficiency of the DPP4 inhibitor used, and in addition can be used as meta data for correlation analysis of the data of the measurement values of the substrate fragments.

Example 3: Measurement of GLP-1

To determine the concentration of human active GLP-1 (= administrated DPP4 substrate) in rat plasma an ELISA from Linco Research Inc., St. Charles, MO, USA, according to the manufacturer's instructions can be performed. This ELISA only detects active GLP-1, e.g. GLP-1 not proteolytically cleaved by a protease. Measurement of GLP-1 can be done using the rat plasma, which is prepared as described in example 1. The measurement values for the active (= not proteolytically cleaved) GLP-1 are useful for determining the efficiency of the DPP4 inhibitor used, for determining the activity of potential second-line proteases, and in addition can be used as meta data for corellation analysis of the data of the measurement values of the substrate fragments.

Example 4: Rat plasma preparation for peptide display

Rat blood samples are consecutively centrifuged at room temperature for 10 min. at 2000 x g and for 15 min. at 2500 x g. The resulting platelet poor plasma is stored at -80°C until analysis. For reduction of the protein load the plasma is subjected to ultrafiltration, which removes the majority of proteins >50 kDa. For this purpose the plasma sample is diluted 1:4 with 8 M guanidinium chloride solution at room temperature and for quality control purposes a peptide standard mixture may be added resulting in a final concentration of 300 to 800 pM of each individual standard peptide added. The peptides added are: Substance P, Somatostatin-14, Neurotensin, Renin Substrate (porcine), Adrenocorticotropic Hormone (ACTH)(1-17), Big Endothelin-1(19-38), ACTH(18-39), beta-Endorphin(6-31), Adrenocorticotropic Hormone (1-24), Calcitonin (human), Insulin B chain oxide (bovine), ACTH(7-38), Pro34-Neuropeptide Y (porcine) and growth hormone-releasing factor (GRF)(1-44). All Peptides or proteins represent the human sequence, if not indicated to the contrary and the numbers in brackets following some of the peptides indicate the amino acid positions of the complete sequence comprising said peptide. Prior to use, the ultrafiltration devices are rinsed with distilled water. Ultrafiltration is done using Amicon Ultra filter devices preferably with a molecular weight cut-off of 50 kDa (Millipore, Bedford, USA), which are centrifuged according to the manufacturers instruction at 4000 x g for 60 min. at room temperature in a swinging bucket rotor. Finally, the pH of the filtrate is adjusted to pH 2 to 3 using concentrated HCl (30% v/v). If the sample (filtrate) is not directly further processed, it is stored at -80°C. A 750 $\mu$l-equivalent of plasma is used per chromatographic run.

Example 5: Liquid chromatography of the samples

The separation of peptides and proteins is done using a Source 5RPC, 4,6 x 150 mm reverse phase chromatography column (Amersham Biosciences Europe GmbH, Freiburg, Germany). Buffer A is 0.06% (v/v) (volume per volume) trifluoroacetic acid (TFA) in distilled water and buffer B is 0.05% (v/v) TFA, and 80 % (v/v) acetonitrile in distilled water. The chromatography takes place at 33°C using a HP 1100 HPLC with a micro flow cell both supplied by Agilent Technologies, Böblingen, Germany. The samples are diluted to 5.5 ml using a 0.1 % (v/v) stock solution of trifluoroacetic acid. Prior to chromatography, the samples are centrifuged at 4°C and 15000 x g for 10 minutes and finally 5 ml of sample are loaded onto the chromatography column representing an equivalent of 750 μl plasma. Buffers A and B are mixed in various ratios during chromatography and in the following only the amount in percentage of buffer B is stated. The amount in percentage of buffer A is 100% minus % buffer B (v/v). The chromatography conditions are as follows:

- 5% buffer B for 1 min
- a linear gradient from 5 to 50% buffer B during the next 44 min.
- a linear gradient from 50 to 100% buffer B during the next 4 min.
- 100% buffer B for the next 4 min.

The flow rate is 500 μl/min. and 96 fractions each containing 0.25 ml are collected during the gradients from 5 to 100% buffer B.

Example 6: Mass spectrometry of the samples

For mass spectrometric analysis, typical positive ion spectra of peptides are measured using a MALDI-TOF (matrix-assisted laser desorption ionization time of flight) mass spectrometer. For MALDI sample preparation alpha-cyano-4-hydroxycinnamic acid is used as matrix and 6-desoxy-1-galactose is used as co-matrix, dissolved in acetonitrile containing 0.1 % TFA. A lyophilized equivalent obtained by reverse phase chromatography corresponding to 7.5 μl plasma spotted onto the mass spectrometric carrier plate (also termed "target") is used to measure the peptides and/or proteins. The fractionated, lyophilized sample is dissolved in 15 μl of a matrix solution. This matrix solution contains 10 g/L α-cyano-4-hydroxycinnamic acid and 10 g/L L(-)fucose dissolved in a solvent mixture consisting of acetonitrile, water, trifluoroacetic acid (TFA) and acetone in a ratio of 49:49:1:1 by volume (v/v). 0.3 μl of this sample solution are transferred to a MALDI carrier plate, and the dried sample is analyzed in a Voyager-DE STR MALDI mass spectrometer from PerSeptive Biosystems. The measurement takes place in linear mode with delayed extraction™. A MALDI-TOF mass spectrometer can be employed to quantify peptides and/or proteins such as, for example, the peptides and proteins described herein if these peptides and/or proteins are present in a concentration which is within the dynamic measurement range of the mass spectrometer, thus avoiding detector saturation.

Example 7: Peptide identification

Detection of concentration differences of individual peptides and/or proteins is achieved by calculation of subtractive peptide display maps and correlation analysis. Selected peptides, which appear in peptide display maps are analyzed by ESI-qTOF sequencing (PE Applied Biosystems, Framingham, USA). Peptide ions are selected in the mass spectrometer on the basis of their specific m/z (mass/charge) values in a manner known to the skilled worker. These selected ions are then fragmented by supplying collision energy (20 - 40 eV) with an collision gas, e.g. helium or nitrogen, and the resulting mass spectrometric fragments of the peptides and/or proteins are detected in the mass spectrometer in an integrated analysis unit, and corresponding m/z values are determined (principle of tandem mass spectrometry). The fragmentation behavior of peptides and/or proteins enables the unambiguous identification of the peptides and/or proteins. The mass-spectrometric analysis can be done for example by Quadrupol-TOF (time of flight) sequencing (QStar-Pulsar model) or ESI-qTOF sequencing (both from PE Applied Biosystems, Framingham, USA). The resulting peptide/protein fragment spectra are detected using nanoSpray in the product ion scan mode (spray voltage 950 V, collision energy 20-40 eV). Up to 200 scans per sample are accumulated. Charge state deconvolution is performed using the Bayesian reconstruct tool of the BioAnalyst program package (PE Applied Biosystems), and deisotoping is achieved by means of the Voyager 5.1 software (PE Applied Biosystems). The mass spectra are saved in a MASCOT generic file format, and submitted to the MASCOT19 database search engine (Matrix Science, London, UK). Searched databases for example can be SwissProt (Version 39.6, www.expasy.ch) and MSDB (Version 010721, EBI, Europe). In addition, this peptide sequencing method allows the identification of amino acid modifications such as phosphorylation, acetylation or hydroxylation.

Example 8: Identification of protease recognition sequences and corresponding proteases

There are numerous tools available to predict protease recognition sequences. These kind of tools usually require as input an amino acid sequence and give as output a prediction, which protease at which position within the amino acid sequence will cleave the given amino acid sequence. These tools can be used to predict proteases which generate distinct proteolytic fragments of a given substrate. Examples of such protease recognition sequence prediction tools among others are: "PeptideCutter" (http://www.expasy.org/tools/peptidecutter/) from the Swiss Institute of Bioinformatics, Basel, Switzerland, the "Protean" tool from the software suite "Lasergene®" of DNAstar Inc., Madison, WI, USA, or the PoPS (Prediction of Protease Specificity, http://pops.csse.monash.edu.au/index.html), Monash University, Clayton in Victoria, Australia (The Fourth IEEE International Conference on Data Mining Brighton, UK, November 01-04, 2004), etc. There are also various neural network approaches to identify protease recognition sequences (IEEE Transact Neural Networks, 2005, 16:263-274) (J Bioinformat and Computat Biol, 2004, 2:511-531) which potentially can be used to predict second-line proteases, based on the sequences of identified proteolytic fragments of said given substrate.

Example 9: In vitro testing of protease recognition sequences

After identification of proteolytic fragments generated by proteolytic activity of second-line proteases and after prediction of putative second-line protease recognition sequences and the corresponding second-line proteases, these second-liene protease recognition sequences and second-line proteases can be tested in vitro. This can be done using various methods know in the art. There are many different assay systems to detect proteolytic activity (John Wiley & Sons, Inc. 2003, Current Protocols in Protein Science, Vol. 3, 21 Peptidases, chapter 21.1 to 21.6) and there are many detection systems and label-systems for various proteases available, for example from Invitrogen GmbH / Molecular Probes, Karlsruhe, Germany; "A Guide to Fluorescent Probes and Labeling Technologies", 10th Edition, chapter 10: Enzyme substrates.

One method to test a protease recognition site of a second-line protease, identified with the methods of the invention is the synthesis of a short peptide comprising the identified second-line protease recognition sequence and bearing at the N- or C-terminus a fluorophore and at the other end of the peptide a corresponding quenching group for said fluorophore. This customized, synthetic substrate can then be used for Fluorescence Resonance Energy Transfer assays (FRET-assays). The synthetic substrate is incubated in vitro with purified or recombinantly expressed proteases predicted to be the second-line proteases cleaving said identified protease recognition sequence. If a protease cleaves said customized synthetic substrate the fluorophore and the quenching group are separated from each other and the fluorophore can be measured using fluorometry. Alternatively such a second-line protease assay can be used to guide the purification of the corresponding second-line protease from the biological sample or fraction thereof in which the second-line protease was found.

Another method to test protease recognition sequences is to prepare a fusion protein comprising the following structure: His-Tag, protease recognition sequence, green fluorescent protein (GFP). This fusion protein is incubated together with the proposed second-line protease. Subsequently the reaction mixture is incubated with a nickel-charged metal-chelate resin. The resin binds any non-cleaved fusion protein and is removed from the reaction mixture. Remaining GFP can be measured in a fluorometer and indicates the proteolytic activity of the putative second-line protease for the protease recognition sequence tested. This assay also can be done in 96-well plates, if magnetic metal-chelate resin is used, which easily can be separated off from the cleaved fusion protein or if small metal-chelate resin spin-columns, assembled in 96-well format, are used. Also this second-line protease assay can be used to guide the purification of the corresponding second-line protease from the biological sample or fraction thereof in which the second-line protease was found.

**Claims**

1. A method of determining whether proteolytic activity for a given substrate is present in a biological sample, the method comprising the steps of:

   a) inhibiting in said biological sample or a fraction thereof the activity of at least one protease known to proteolytically cleave said given substrate,
   b) allowing the proteases present in said biological sample or said fraction thereof to exert proteolytic activity on said given substrate in said biological sample or said fraction thereof, and
   c) analysing said given substrate and/or proteolytic fragments thereof generated in step b).

2. A method of identifying at least one sequence in a substrate which is subject to cleavage by proteases present in a biological sample, the method comprising the steps of:

   a) inhibiting in said biological sample or a fraction thereof the activity of at least one protease known to proteolytically cleave said given substrate,
   b) allowing the proteases present in said biological sample or said fraction thereof to exert proteolytic activity on said given substrate in said biological sample or said fraction thereof, and
   c) analysing said given substrate and/or proteolytic fragments thereof generated in step b).

3. The method of claim 1 or 2, wherein said given substrate on which proteolytic activity in step (b) is allowed to be exerted is naturally present in said biological sample and/or has been added to said biological sample or said fraction thereof.

4. A method of determining whether a given sequence is subject to cleavage by proteases present in a biological sample, the method comprising the steps of:

   a) inhibiting in said biological sample or a fraction thereof the activity of at least one protease known to proteolytically cleave a substrate comprising said given sequence or a variant thereof,
   b) allowing the proteases present in said biological sample or said fraction thereof to exert proteolytic activity on a substrate comprising said given sequence in said biological sample or said fraction thereof, and
   c) analysing said substrate comprising said given sequence and/or proteolytic fragments thereof generated in step b).

5. The method of any one of claims 1 to 4, wherein said substrate on which proteolytic activity in step (b) is allowed to be exerted comprises at least one label, preferably selected from the group consisting of stable isotope-labels, radioactive isotope-labels, dye-labels, fluorescent labels, FRET-labels, particle-labels, ITRAQ-labels.

6. The method of any one of claims 1 to 5, wherein step (a) comprises an inhibition of the activity of at least one first-line protease for said given substrate.

7. The method of any one of claims 1 to 6, wherein said proteolytic activity in step (b) is exerted at least in part by at least one second-line protease.

8. The method of claim 6 or 7, wherein said at least one first-line protease and/or said at least one second-line protease are naturally present in said biological sample.

9. The method of any one of claims 1 to 8, wherein said inhibition of the activity of said at least one protease is achieved by means of at least one protease inhibitor for said at least one protease.

10. The method of any one of claims 1 to 9, wherein said step (c) comprises a qualitative and/or quantitative analysis of said substrate and/or said proteolytic fragments thereof.

11. The method of any one of claims 1 to 10, wherein said step (c) includes a determination of at least one full or partial sequence of at least one of said proteolytic fragments, wherein said at least one full or partial sequence of said proteolytic fragments is preferably used to determine the sequence of at least one protease recognition sequence for at least one of the proteases present in said biological sample.

12. The method of claim 11, wherein the sequence of said at least one protease recognition sequence is used to identify at least one protease responsible for cleavage of said substrate.

13. The method of any one of claims 1 to 12, further comprising the step of comparing the results obtained in said step (c) with a reference sample.

14. The method of any one of claims 1 to 13, further comprising the step of:

comparing the results obtained in said step (c) with the results obtained from (i) analysing the substrate and/or proteolytic fragments thereof prior to step (a) in said biological sample or fraction thereof or a corresponding biological sample or fraction thereof and/or (ii) analysing the substrate and/or proteolytic fragments thereof in a corresponding biological sample or fraction thereof in which the activity of at least one of said at least one protease is not inhibited and/or (iii) analysing the substrate and/or proteolytic fragments thereof in a corresponding biological sample or fraction thereof in which the activity of at least one of said at least one protease is inhibited to a different extent and/or (iv) analysing the substrate and/or proteolytic fragments thereof in a corresponding biological sample or fraction thereof in which the activity of at least one of said at least one protease is inhibited by different means and/or (v) analysing the substrate and/or the proteolytic fragments thereof in a corresponding biological sample or fraction thereof in which the activity of at least one of said at least one protease is inhibited for a different period of time.

15. The method of any one of claims 1 to 14, wherein step (a) or steps (a) and (b) take place within a living organism.

16. The method of any one of claims 1 to 15, wherein step (a) or steps (a) and (b) take place within a living organism and said method further comprises the step of:

comparing the results obtained in step (c) with the results obtained from at least one further living organism.

17. The method of claim 16, wherein in said at least one further living organism (i) the activity of at least one of said at least one protease is not inhibited and/or (ii) the activity of at least one of said at least one protease is inhibited to a different extent and/or (iii) the activity of at least one of said at least one protease is inhibited by different means and/or (iv) the activity of at least one of said at least one protease is inhibited for a different period of time.

18. The method of claim 16 or 17, wherein said living organism and said at least one further living organism have been subjected or are subjected to different environmental conditions and/or are of different age, sex, weight or health condition.

19. The method of any one of claims 15 to 18, wherein said living organism and/or said at least one further living organism is afflicted with a disease.

20. The method of any one of claims 15 to 19, wherein said living organism and/or said at least one further living organism is a multicellular organism, preferably a mammal.

21. The method of any one of claims 15 to 20, wherein said inhibition of the activity of said at least one protease is achieved by means of at least one protease inhibitor for said at least one protease and said at least one protease inhibitor is delivered to said living organism and/or said at least one further living organism.

22. The method of any one of claims 6 to 21, wherein said at least one first-line protease and/or said at least one second line-protease comprise at least one protease selected from the group consisting of aspartic peptidases, cysteine peptidases, glutamic peptidases, metallo peptidases, serine peptidases, threonine peptidases, amino peptidases, carboxypeptidases, endopeptidases and exopeptidases.

23. The method of claim 22, wherein said first-line protease is dipeptidyl peptidase 4 (DPP4).

24. The method of claim 23, wherein said given substrate is glucagon-like peptide 1 (GLP-1).

25. The method of any one of claims 1 to 24, wherein said step (c) comprises an analysis of the entire biological sample or the entire fraction thereof or of a fraction of said biological sample or said fraction thereof.

26. The method of any one of claims 1 to 25, wherein said step (c) comprises a determination of the relative or absolute quantity of said substrate and/or said proteolytic fragments thereof.

27. The method of any one of claims 1 to 26, wherein said step (c) is performed using mass spectrometric methods optionally in combination with separation methods for said substrate and/or proteolytic fragments thereof.

28. The method of claim 27, wherein said separation methods are selected from the group consisting of gel electrophoresis, liquid chromatography, gas chromatography, capillary chromatography, thin layer chromatography, mass spectrometry, precipitation techniques, liquid phase extraction techniques, filtration and other molecular size discriminating techniques.

29. The method of any one of claim 27 or 28, wherein said mass spectrometric methods are selected from the group consisting of MALDI mass spectrometry, ESI mass spectrometry, SELDI mass spectrometry, FAB mass spectrometry and MS/MS mass spectrometry.

30. The method of any one of claims 1 to 29, wherein said biological sample is selected from the group consisting of blood, serum, plasma, urine, tear fluid, saliva, synovia, liquor, bronchial aspirate, sputum, lymph, tissue extracts, cell extracts and cell culture medium.

**Figure 1**

# A

Protease DPP4    Protease Y                    Starting conc.
     ↓              ↓                           of substrate

1. HAEGTFTSDVSSYLEGQAAKEFIAWLVKGR   ▬

2. HAEGTFTSDVSSYLEGQAAKEFIAWLVKGR   ■

3. HAEGTFTSDVSSYLEGQAAKEFIAWLVKGR   ■

4. HAEGTFTSDVSSYLEGQAAKEFIAWLVKGR   ▬

# B

Combined conc.
of fragments

Protease Y Generated fragments

5. HAEGTFTSDVSSY          ▬

6. HAEGTFTSDVSSY          ■▬

7. HAEGTFTSDVSSY          ■

8. LEGQAAKEFIAWLVKGR      ▬▬▬

9. LEGQAAKEFIAWLVKGR      ▬

# C

Combined conc.
of fragments

Protease DPP4 Generated fragments

10. HA                              ▬▬

11. HA                              ■■

12. EGTFTSDVSSYLEGQAAKEFIAWLVKGR   ▬■

13. EGTFTSDVSSYLEGQAAKEFIAWLVKGR   ■

14. EGTFTSDVSSYLEGQAAKEFIAWLVKGR   ▬

# Figure 2

**European Patent Office**

## PARTIAL EUROPEAN SEARCH REPORT

Application Number

which under Rule 45 of the European Patent Convention shall be considered, for the purposes of subsequent proceedings, as the European search report

EP 05 01 7523

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | KIM KWANG-ROK ET AL: "KR-62436, 6-{2-[2-(5-cyano-4,5-dihydropyrazol-1-yl)-2-oxoethylamino]e thylamino}nicotinonitrile, is a novel dipeptidyl peptidase-IV (DPP-IV) inhibitor with anti-hyperglycemic activity." EUROPEAN JOURNAL OF PHARMACOLOGY. 25 JUL 2005, vol. 518, no. 1, 25 July 2005 (2005-07-25), pages 63-70, XP002348027 ISSN: 0014-2999 * the whole document * <br><br> ----- <br><br> -/-- | 1-6,8-30 | C12Q1/37 |

TECHNICAL FIELDS SEARCHED (Int.Cl.7)

C12Q

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 20 October 2005 | Rosin, O |

European Patent
Office

**PARTIAL EUROPEAN SEARCH REPORT**

Application Number

EP 05 01 7523

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | PAULY R P ET AL: "Investigation of glucose-dependent insulinotropic polypeptide-(1-42) and glucagon-like peptide-1-(7-36) degradation in vitro by dipeptidyl peptidase IV using matrix-assisted laser desorption/ionization-time of flight mass spectrometry. A novel kinetic approach." THE JOURNAL OF BIOLOGICAL CHEMISTRY. 20 SEP 1996, vol. 271, no. 38, 20 September 1996 (1996-09-20), pages 23222-23229, XP002348028 ISSN: 0021-9258 * the whole document * ----- | 1,7 | |
| X | PAULY R P ET AL: "Improved glucose tolerance in rats treated with the dipeptidyl peptidase IV (CD26) inhibitor Ile-thiazolidide." METABOLISM: CLINICAL AND EXPERIMENTAL. MAR 1999, vol. 48, no. 3, March 1999 (1999-03), pages 385-389, XP002348029 ISSN: 0026-0495 * the whole document * ----- | 1-6, 8-18, 20-28,30 | TECHNICAL FIELDS SEARCHED (Int.Cl.7) |
| X | DEMUTH HANS-ULRICH ET AL: "Type 2 diabetes--therapy with dipeptidyl peptidase IV inhibitors." BIOCHIMICA ET BIOPHYSICA ACTA. 1 AUG 2005, vol. 1751, no. 1, 1 August 2005 (2005-08-01), pages 33-44, XP002348030 ISSN: 0006-3002 * the whole document * ----- | 1,3 | |

EPO FORM 1503 03.82 (P04C10)

**European Patent Office**

**INCOMPLETE SEARCH
SHEET C**

Application Number

EP 05 01 7523

Although claims 15-21 are directed to a method of treatment of the human/animal body (Article 52(4) EPC), the search has been carried out and based on the alleged effects of the compound/composition.

-----

Reason for the limitation of the search (non-patentable invention(s)):

Article 52 (4) EPC - Diagnostic method practised on the human or animal body

**EP 1 795 606 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1553515 A1

### Non-patent literature cited in the description

- **LEUNG et al.** *J. Med. Chem.,* 2000, vol. 43, 305-41
- **SOUTHAN.** *Drug Discov. Today,* 2001, vol. 6, 681-8
- **SOUTHAN.** *FEBS Lett.,* 2001, vol. 498, 214-8
- *PNAS,* 1992, vol. 89, 9367
- *Acc Chem Res,* 1993, vol. 26, 266
- *Science,* 1993, vol. 261, 1303
- *J Am Chem Soc,* 1992, vol. 114, 10672
- *J Am Chem Soc,* 1992, vol. 114, 6570
- *J Med Chem,* 1999, vol. 42, 1041-52
- *The Fourth IEEE International Conference on Data Mining Brighton*
- *IEEE Transact Neural Networks,* 2005, vol. 16, 263-274
- *J Bioinformat and Computat Biol,* 2004, vol. 2, 511-531
- Current Protocols in Protein Science. John Wiley & Sons, Inc, 2003, vol. 3
- A Guide to Fluorescent Probes and Labeling Technologies. Molecular Probes. Invitrogen GmbH